# EUROPEAN PATENT SPECIFICATION

(11) **EP 1 337 528 B1**
(45) Date of publication and mention of the grant of the patent: **04.08.2004**
(21) Application number: 01983737.6
(22) Date of filing: 19.11.2001
(51) Int. Cl.: C07D 409/12, C07D 333/56, A61K 31/497, A61P 25/24

(54) **BENZOTHIOPHENE DERIVATIVE COMPOUNDS, PROCESS OF PREPARATION AND USE THEREOF**
BENZOTHIOPHENDERIVATE, VERHAHREN ZU IHRER HERSTELLUNG UND IHRE VERWENDUNG
COMPOSES DERIVES DE BENZOTHIOPHENE, LEUR PROCEDE DE PREPARATION ET D'UTILISATION

(30) Priority: 29.11.2000 ES 200002914
(43) Date of publication of application: 27.08.2003
(73) Proprietor: LABORATORIOS VITA, S.A., 08970 Sant Joan Despi (Barcelona) (ES)
(72) Inventor: DEL CASTILLO NIETO, Juan Carlos, E-08025 Barcelona (ES); LASHERAS ALDAZ, Berta Esperanza, E-Baranain (ES); MONGE VEGA, Antonio, E-Cizur-Menor (ES); MOURELLE MANCINI, Marisabel, E-08028 Barcelona (ES); DEL RIO ZAMBRANA, Joaquin, E-Madrid (ES)
(74) Representative: Ponti Sales, Adelaida
(86) International application number: PCT/IB2001/002211
(87) International publication number: WO 2002/044170

(56) References cited:
- EP-A- 0 546 583
- EP-A- 0 574 313
- WO-A-96/15792
- WO-A-96/16052
- WO-A-99/02516

## Description

### Field of the invention

This invention relates to new benzothiophene derivative compounds, their salts, solvates, optical isomers, geometric isomers and polymorphs.

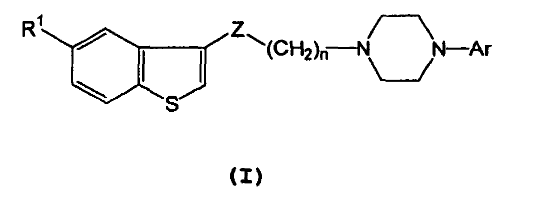

This invention also relates to a process for the preparation of said benzothiophene derivative compounds and their corresponding pharmaceutical compositions and their use in the preparation of these pharmaceutical compositions for the treatment of neurological disorders.

In particular, the compounds of this invention are applied to the treatment of anxiety and/or depression, due to their being antidepressant agents with dual activity: inhibition of serotonin reuptake and affinity for the 5-HT_{1A} receptor.

### Background of the invention

Drugs for the treatment of depression have existed for over 30 years. Both the first inhibitor of monoamine oxydase (MAO inhibitor), iproniazide, and the first tricyclic antidepressant (TCA), imipramine, were introduced into the market at the end of the 1950s. The second-generation antidepressants were a considerable improvement over the traditional tricyclic antidepressants and the irreversible and non-specific MAO inhibitors. Despite this, they still had side-effects and, more importantly, the latency period up to manifestation of the therapeutic effect remained too long for the treatment to be considered optimal.

The last class of antidepressants introduced in the market are the selective serotonin reuptake inhibitors, notable amongst them being the following: fluoxetine (Lilly ES 433720), paroxetine (Ferrosan, ES 422734) and sertraline (Pfizer, ES 496443). This class of compounds presents a high degree of structural diversity when compared with other types of serotonin reuptake inhibitors, such as the tricyclic antidepressants. In spite of their structural diversity, these compounds show high selectivity for the serotonin receptor. Their union with α and β adrenergic, dopaminergic, histamine and muscarinic receptors is not in fact very significant. It is postulated that this may be due to a considerable structural similarity to the pharmacophore which is responsible for their specificity to and relative affinity with the corresponding serotonin receptor.

Among the most frequent adverse effects of the serotonin reuptake inhibitors are those related with gastrointestinal alterations. Most of them also cause inhibition of the hepatic metabolism of other medicines, with the corresponding pharmacodynamic interactions, while also presenting retarded onset of their antidepressant action.

In view of this background it was felt necessary to continue research in order to create a third generation of antidepressants. The four points that an antidepressant must comply with for it to be considered third-generation are:
1. Faster action.
2. Broader efficacy.
3. Fewer side-effects.
4. Safer in overdose situations.

Of these four points, the first is the one that poses the greatest challenge for research into antidepressants, as patients suffering from depression are harmed by the fact that the drug does not begin to exercise its therapeutic effect until after several weeks from the start of the treatment.

The cause of the delay in remission of the illness following a course of treatment with monoamine reuptake inhibitors seems to be due to a process of desensitisation of the presynaptic 5-HT_{1A} receptors, which means that until such desensitisation occurs the seratonergic tone is diminished.

From all this it emerges that an antidepressant treatment which, in addition to inhibition of serotonin reuptake, also involves rapid blocking or desensitisation of the somatodendritic 5-HT_{1A} autoreceptors would increase the antidepressant activity by allowing the concentration of serotonin in serotoninergic endings to rise rapidly. In this respect, it has been proposed that serotonin reuptake inhibitors be administered together with selective antagonists of the 5-HT_{1A} receptors, such as pindolol (Artigas F. and col. *Arch. Gen. Psychiatry,* **51,** 248-251 (1994); Blier P. and col., *J. Clin. Pharmacol*. **15,** 217-222 (1995)), in order to promote a more rapid onset of this antidepressant effect. This theory has led researchers to hypothesise that the addition of products that block 5-HT_{1A} autoreceptors might prevent the onset of this negative feedback system and enhance the effect of the serotonin reuptake inhibitors.

In a patent from Lilly (EP 687472) a boosting of the effect of the serotonin reuptake inhibitors is claimed by increasing the availability of certain cerebral neurotransmitters (among them serotonin) by combining the serotonin reuptake inhibitors with selective antagonists of the 5-HT_{1A} receptors.

It would thus be desirable to have compounds which present this dual activity, that is, serotonin reuptake inhibitors with affinity towards the 5-HT_{1A} receptors.

New benzothiophene derivative compounds have been discovered recently which do have this dual activity and which are therefore potentially useful for treatment of depressive states.

In the bibliography products have been claimed which have some similarities with those described herein.

On the one hand, the patents WO 9616052 and WO 9615792 describe products of general formula: 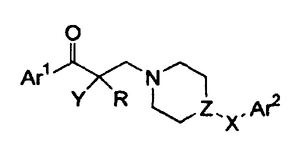 where Z is N or CH, and Ar¹ can be a benzothiophene ring. In these compounds the aromatic ring (Ar²) is not attached directly to the piperazine ring, with Z=N, but instead through a spacer X (CH₂, CO, etc.).

On the other hand, patent DE 2360545 describes piperazines which include the compound: 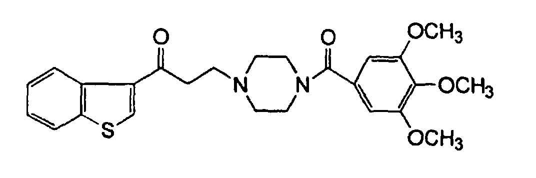 In this benzothiophene derivative, as in the previous cases, the piperazine ring is not attached directly to the aromatic ring, but rather through a spacer element, in this case a C=O group.

Patent WO 9902516, from the present applicant, describes thiophenes and benzothiophenes of the following general formula: 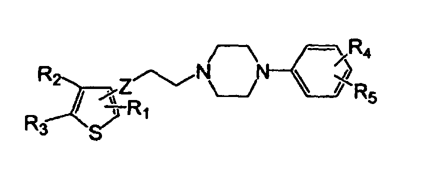 Where R₄ and R₅ represent independently various substituents or can form together a benzene ring fused onto the phenyl ring.

This invention proposes the disclosure of new benzothiophene derivative compounds with greater inhibitory action on reuptake of serotonin, in addition to retaining high affinity towards the 5-HT_{1A} receptor.

### Description of the invention

The object of this invention are new benzothiophene derivatives of general formula (I): 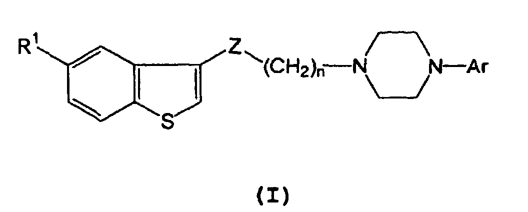 where:
- n is 1, 2 or 3;
- Z is C=O or -CHOH;
- R¹ is H, alkyl C₁-C₆, halogen, -OR², nitro, cyano, -NR³R⁴, -COR², -CO₂R², -O-COR², -SO₂NR³R⁴, -SO₂R², -SR², or -CONR³R⁴;
- R² is H, alkyl C₁-C₆ or phenyl;
- R³ and R⁴ are independent from each other and stand for H, alkyl C₁-C₆ or phenyl, or else R³ together with R⁴ form a morpholine, thiomorpholine or piperazine ring;
- Ar is an optionally substituted bicylic system formed by a benzocondensed heterocyclic ring with 5, 6 or 7 ring atoms, saturated or unsaturated and containing 1, 2 or 3 hetero-atoms selected from N, O and S;
a pharmaceutically acceptable salt or solvate, or any geometric isomer, optical isomer or polymorph thereof.

In this invention, the term "a pharmaceutically acceptable solvate" is taken to mean a hydrate or solvate of a C₁-C₆ alcohol.

In this invention, the term "pharmacologically acceptable salts" includes the acid-addition salts formed with inorganic and organic acids, such as hydrochlorides, hydrobromides, sulphates, nitrates, phosphates, formiates, mesylates, citrates, benzoates, fumarates, maleates, lactates and succinates, among others. When a salt is formed from a compound of formula I with a dicarboxylic acid, such as succinic acid, the salt may contain between one and two moles of the compound of formula (I) per mole of acid.

The term "optical isomers" includes the optical isomers (R and S) when Z stands for -CHOH together with its enantiomeric mixtures.

Surprisingly, the compounds of the invention have an inhibitory effect on serotonin reuptake greater than that of the compounds described in WO 9902516, the prior art closest to the application, while at the same time retaining values of the same order of affinity towards the 5-HT_{1A} receptor.

Advantageously, compounds of general formula (I) are preferable, in which the number of hetero-atoms is 1 or 2.

Likewise, preferably Ar is: 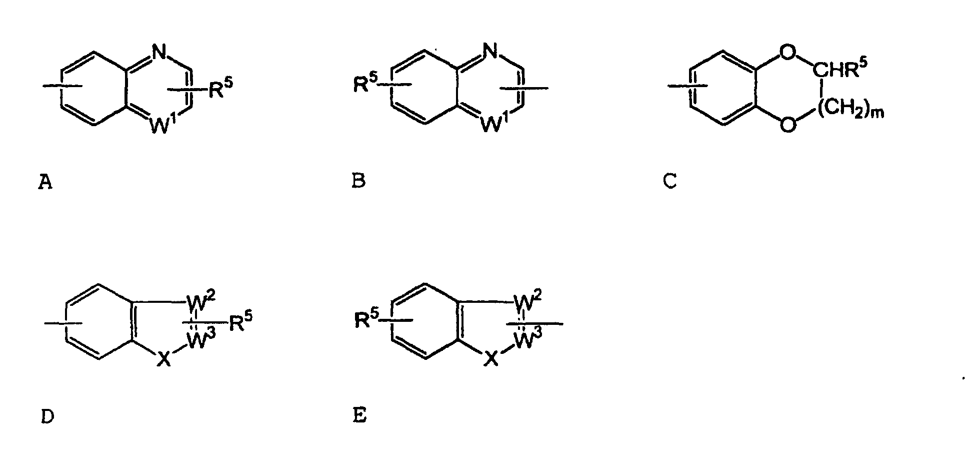 where:
- m is 0, 1 or 2;
- W¹ is CH or N;
- W² and W³ are independently, the same or different, CH, CH₂ or N;
- X is N, O or S;
- R⁵ is H, alkyl C₁-C₆, alkyl C₁-C₆ substituted by a hydroxy or halogen group, halogen, -OR⁶, nitro, cyano, -NR⁷R⁸, -COR⁶, -CO₂R⁶, -SO₂NR⁷R⁸, -SO₂R⁶, -SR⁶, or -CONR⁷R⁸ ;
- R⁶ is H, alkyl C₁-C₆ or phenyl;
- R⁷ and R⁸ are independent of each other and stand for H, alkyl C₁-C₆ or phenyl, or else R⁷ together with R⁸ form a morpholine, thiomorpholine or piperazine ring.

Preferably n is 2.

Preferably R¹ is halogen, NO₂, OH, H or CH₃.

The preferable compounds are selected from the following:
- 1-(5-nitrobenzo[b]thiophen-3-yl)-3-[4-(2,3-dihydro-1,4-benzodioxyn-5-yl)piperazin-1-yl]propan-1-one
- 1-(5-fluorobenzo[b]thiophen-3-yl)-3-[4-(quinol-8-yl) piperazin-1-yl]propan-1-ol
- 1-(5-fluorobenzo[b]thiophen-3-yl)-3-[4-(2,3-dihydro-1,4-benzodioxyn-5-yl)piperazin-1-yl]propan-1-ol
- 1-(5-fluorobenzo[b]thiophen-3-yl)-3-[4-(indol-4-yl)piperazin-1-yl]propan-1-ol
- 1-(5-fluorobenzo[b]thiophen-3-yl)-3-[4-(quinaldin-8-yl)piperazin-1-yl]propan-1-ol
- 1-(benzo[b]thiophen-3-yl)-3-[4-(quinol-8-yl)piperazin-1-yl]propan-1-ol hydrochloride
- 1-(benzo[b]thiophen-3-yl)-3-[9-(indol-4-yl)piperazin-1-yl]propan-1-ol hydrochloride
- 1-(benzo[b]thiophen-3-yl)-3-[4-(quinaldin-8-yl)piperazin-1-yl]propan-1-ol hydrochloride
- 1-(benzo[b]thiophen-3-yl)-3-[4-(quinol-5-yl)piperazin-1-yl]propan-1-ol
- 1-(5-nitrobenzo[b]thiophen-3-yl)-3-[4-(2,3-dihydro-1,4-benzodioxyn-5-yl)piperazin-1-yl]propan-1-ol
- 1-(benzo[b]thiophen-3-yl)-3-[4-(quinol-2-yl)piperazin-1-yl]propan-1-ol
- 1-(benzo[b]thiophen-3-yl)-3-[4-(quinol-4-yl)piperazin-1-yl]propan-1-ol hydrochloride
- 1-(benzo[b]thiophen-3-yl)-3-[9-(quinol-6-yl)piperazin-1-yl]propan-1-ol
- 1-(5-hydroxybenzo[b]thiophen-3-yl)-3-[4-(2,3-dihydro-1,4-benzodioxyn-5-yl)piperazin-1-yl]propan-1-ol

Also object of this invention is a process for preparing the compounds of general formula (I), a pharmaceutically acceptable salt or solvate thereof.

The process for preparing a compound of general formula (I), a pharmaceutically acceptable salt or solvate thereof, is characterised in that it comprises:
a substitution reaction of the Hal group of a compound of general formula (II) with a suitable piperazine of general formula (III): 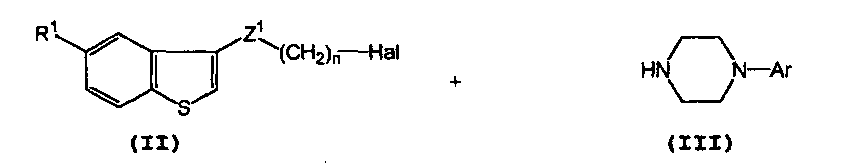 where:
   - n, R¹ and Ar have the meaning defined above,
   - Hal represents a halogen,
   - Z¹ represents C=O, or CHOR⁹ y
   - R⁹ represents H or an alcohols protecting group; in the presence of an inert solvent and an organic or inorganic base;
and then, if required, carrying out one or more of the following optional steps:
   - Converting a compound of general formula (I) into another compound of general formula (I);
   - Eliminating any protecting group;
   - Preparing a pharmacologically acceptable salt of a compound of general formula (I) and/or a pharmacologically acceptable solvate thereof.

On the basis of said process compounds of general formula (Ia) can be made, where Z is a ketone C=O, and compounds of general formula (Ib), where Z = CHOH.

The reaction is carried out in an inert solvent and in the presence of a base. Advantageously, said inert solvent is selected from tetrahydrofuran, dichloromethane, toluene or dimethylformamide and said organic or inorganic base is selected from potassium carbonate, potassium bicarbonate, triethylamine and diisopropylamine.

The compounds of formula (II) in which Z¹ = C=O, can in turn be obtained according to methods described in the bibliography:
- When n=1, starting from acetylbenzo[b]thiophene optionally substituted (Majumdar, K.C. and Thiagarajan, B.S.; *Int. J. Sulphur Chem.;* A; **2**; n°2, (1972) 93-103)
- When n= 2 or 3, by reaction of benzothiophene optionally substituted with haloalkyl halide ( Hal-(CH₂)ₙ-C(O)Hal ) in the presence of aluminium trichloride (WO 9902516).

The compounds of formula (II) in which Z¹= CHOR⁹ are made by reduction of the corresponding ketones, by the usual methods described in the bibliography for reduction of ketones, followed optionally by an alcohol protection stage.

The piperazines of formula III can be prepared in accordance with methods described in the bibliography. For example:
- The 1-(2-quinolil)-piperazine (Quipazine) is made using the method described in US 4487773.
- 1-(3-quinolil)-piperazine is made as described in patent EP 802173.
- 1-(4-quinolil)-piperazine is made using the method described in M.D. Abel *et al. J.Het.Chem.* (1996) 415.
- 1-(5-quinolil)-piperazine is made using the method described in EP 279598.
- 1-(6-quinolil)-piperazine, th 1-(8-quinolil)-piperazine and the 1-(2-methylquinolil)-piperazine are made in a similar way to the 1-(5-quinolil)-piperazine, starting with 6-aminoquinoline, 8-aminoquinoline and 2-methyl-8-aminoquinoline, respectively.
- Indol-4-yl-piperazine is made using the method described in WO 9533725.
- 4-(1-piperazinil)indol-2-ethyl carboxylate is made using a method similar to that described in WO 9415919 for the corresponding metal ester. Similarly, the 6-(1-piperazinil)indol-2-ethyl carboxylate is made by starting with 6-aminoindol-2-ethyl carboxylate.
- 1-(2,3-dihydro-1,4-benzodioxy-5-yl)-piperazine is made using the method described by J.L.Peglion *et al*. *(J.Med.Chem.,* (1995) **38**.4044-4055).
- 1-(2,3-dihydro-1,4-benzodioxy-6-yl)-piperazine is made in a similar way to the previous one, starting from 6-amino-2,3-dihydro-1,4-benzodioxane.
- The 1-(3,4-dihydro-2H[1.5]-benzodioxepin-6-yl)-piperazine is made using the method described by Wijngaarden *J. et al. J.Med.Chem.* (1988) **31,** 1934-1940.
- The 1-(benzo[1.3]dioxol-4-yl)-piperazine is made in a similar way to the piperazine described above, from 4-benzo[1.3]dioxol-4-ylamine.

In addition to preparing them using the general method described above, the alcohols of general formula (Ib) can also be prepared by reduction of the ketones of general formula (Ia).

The alcohol derivatives of general formula (Ib), 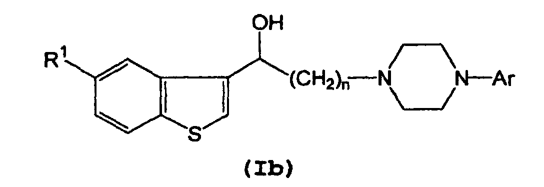 can be made by reduction of the ketones (Ia): 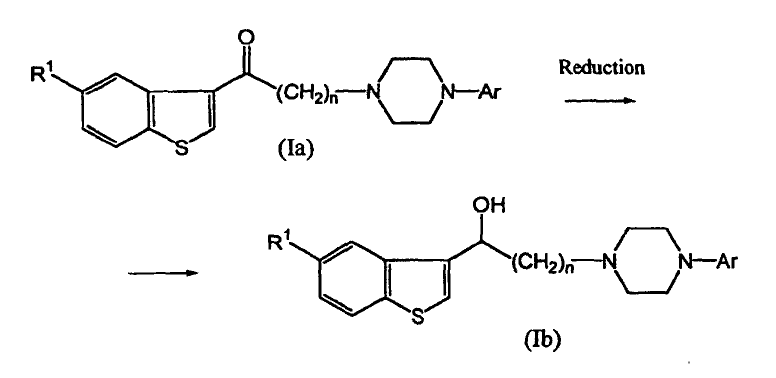 where n, R¹ and Ar have the meaning defined above,
a) in the presence of a reducing agent at a temperature between -20°C and 200°C and in an inert solvent; or
b) by catalytic hydrogenation; and then, if required, one or more of the following optional steps may be carried out:
   - Converting a compound of formula (Ib) into another compound of formula (Ib);
   - Eliminating any protecting group;
   - Preparing a pharmacologically acceptable salt of a compound of formula (Ib) and/or a pharmacologically acceptable solvate thereof.

Preferably, said reducing agent is a metal hydride such as sodium borohydride and said solvent is an alcohol, preferably methyl or ethyl alcohol.

Also preferably, the reaction temperature is between -20°C and the reflux temperature of the alcohol, advantageously at the reflux temperature.

Likewise, the reduction reaction can be accompanied in some cases, depending on the value of R¹, by transesterification or hydrolysis reactions.

A third process for preparing compounds of general formula (I) consists in transformation, using the methods described in the literature, of a substituent in a compound of general formula (I) into a different substituent, thus providing a different compound corresponding structurally to the compound of general formula (I).

One example of this type of transformation consists in the reduction of an aromatic NO₂ group, by the methods described in the literature, to an amine group, as specified below.

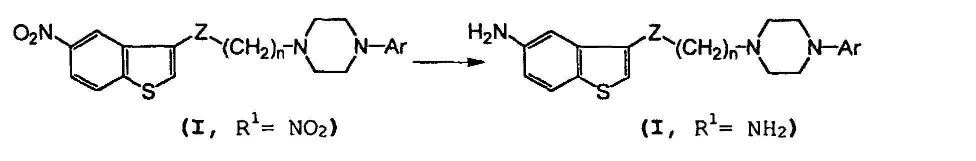

The reduction of the NO₂ group can be carried out by means of hydrogenation in the presence of a catalyst, such as Pd/C or Ni Raney.

The reduction reaction of the NO₂ group, where Z= CHOH, can also be carried out with hydracin and Ni Raney.

Where appropriate, the isomeric forms of the compounds of general formula (I) or their pharmaceutically acceptables salts can be prepared as individual isomers using the conventional chemical methods.

The compounds of general formula (I) can be administered *per se* or in pharmaceutical compositions that also include pharmaceutically acceptable excipients.

Also objects of this invention, therefore, are compositions which comprise a compound of general formula (I), a pharmaceutically acceptable salt or solvate, or any geometric isomer, optical isomer or polymorph thereof in a therapeutically active amount and a suitable amount of a pharmacologically acceptable carrier for use in the treatment of neurological disorders.

The compositions provided by this invention are preferably for oral administration, though they can also be adapted to other forms of administration such as injectables or by percutaneous absorption.

Tablets and capsules are preferred forms of administration, though other forms can be used, such as powders in sachets.

In accordance with conventional pharmaceutical practice, the excipients may include diluents, disintegrators, wetting agents, lubricants, colorants, aromas or other conventional adjuvants.

Typical excipients include, for example, microcrystalline cellulose, starch, polyvinyl pyrrolidone, magnesium stearate or lauryl sodium sulphate.

The compounds of general formula (I) act as serotonin reuptake inhibitors and show affinity towards the 5-HT_{1A} receptor and, thereofore, are potentially useful in the treatment of neurological disorders such as depression, psychosis, anxiety, panic attacks, obsessive-compulsive disorders and nutrition disorders.

In accordance with this, also object of this invention is a compound of general formula (I), a pharmaceutically acceptable salt or solvate thereof, or any geometric isomer, optical isomer or polymorph thereof for use as an inhibitor of serotonin reuptake and antagonist or agonist of the 5-HT_{1A} receptor.

Also object of this invention is the utilization of a compound of general formula (I), a pharmaceutically acceptable salt or solvate thereof, or any geometric isomer, optical isomer or polymorph thereof for manufacturing a medicine for the treatment of neurological disorders such as depression, psychosis, anxiety, panic attacks, obsessive-compulsive disorders and dietary disorders.

### EXPERIMENTAL PART

Outlined below, for the purpose of non-restrictive explanation, are the following examples:

### EXAMPLES OF SYNTHESIS

### Method A

### Example 1:

### 1-(5-fluorobenzo[b]thiophen-3-yl)-3-[4-(quinol-8-yl)pipera-zin-1-yl]propan-1-one hydrochloride

1.7 g of (5-fluorobenzo[b]thiophen-3-yl)-3-chloropropan-1-one (7 x 10⁻³ mol) made as described in EN 2128266, are made to react with 1.5 g of 1-(8-quinolil)-piperazine (7 x 10⁻³ mol) and 0.97 g (7 x 10⁻³ mol) of potassium bicarbonate in 50 ml of THF. Following 24 h of reflux agitation the THF is eliminated in the rotary evaporator and the residue is chromatographed on silica gel. Extraction with CH₂Cl₂/EtOH 95/5 provides a product that solidifies by agitation in 2-propanol.

Thus is made 1.4 g (yield 50%) of a solid that is dissolved in acetone and the hydrochloride is precipitated by treatment with HCl_{(c)}.

M.p.: 215-217°C

IR (KBr) (cm⁻¹): 1673 (s, C=O).

¹H-NMR (DMSO-d₆, 200 MHz) δ (ppm): 3.38 (b.s., 12H, CH₂-s); 7.19 (d, 1H, H₃', J= 7.02); 7.36-7.60 (m, 4H, H₅'+ H₆'+ H₇'+ H₆); 8.18 (c, 2H, H₄+ H₇); 8.33 (d, 1H, H₄', J= 8.27); 8.88 (d, 1H, H₂', J= 2.95); 9.22 (s, 1H, H₂); 11.34 (b.s., 1H, HCl).

MS-DIP (70 eV) m/z (% Abundance): 255 (1), 213 (11), 206 (51), 179 (100), 157 (23).

Proceeding as described in Example 1 and replacing the 1-(quinolil)-piperazine with the corresponding piperazine the following products are made:

### Example 2:

### 1-(5-fluorobenzo[b]thiophen -3-yl)-3-[4-(quinaldin-8-yl)piperazin-1-yl]propan-1-one dihydrochloride

M.p.: 230-232°C

IR (KBr) (cm⁻¹): 1662 (s, C=O).

¹H-NMR (DMSO-d₆, 200 MHz) δ (ppm): 2.63 (s, 3H, CH₃); 2.72 (d, 4H, N¹(CH₂)₂); 2.82 (d, 2H, COCH₂C*H*_{*2*}); 3.29 (t, 6H, N⁴(CH₂)₂+COC*H*_{*2*}); 7.36-7.88 (m, 5H, quinaldine); 8.16-8.28 (m, 1H, H₆); 8.32 (dd, 1H, H₇, J_{F7}= 5.84); 8.71 (d, 1H, H₄, J_{F4}= 7.94); 9.27 (s, 1H, H₂); 11.56 (b.s., 2H, HCl).

MS-DIP (70 eV) m/z (% Abundance): 206 (52), 179 (100), 171 (45), 143 (40).

### Example 3:

### 1-(5-fluorobenzo[b]thiophen-3-yl)-3-[4-(2,3-dihydro-1,4-benzodioxyn-5-yl)piperazin-1-yl]propan-1-one hydrochloride

M.p.: 191-193°C

IR (KBr) (cm⁻¹): 1668 (s, C=O).

¹H-NMR (DMSO-d₆, 200 MHz) δ (ppm): 3.05 (t, 4H, N¹(CH₂)₂); 3.23 (t, 2H, COCH₂C*H*_{*2*}); 3.60 (t, 4H, N⁴(CH₂)₂) 3.72 (t, 2H, COC*H*_{*2*}); 4.24 (b.s., 4H, O(CH₂)₂); 6.55 (d, 2H, H₆'+H₈'); 6.76 (t, 1H, H₇'); 7.40 (ddd, 1H, H₆, J₆₇= 8.94, J₄₆= 2.5); 8.18 (c, 1H, H₇); 8.30 (dd, 1H, H₄, J_{F4}= 10.7); 9.20 (s, 1H, H₂); 10.83 (b.s., 1H, HCl).

MS-DIP (70 eV) m/z (% Abundance): 247 (1), 233 (2), 220 (29), 206 (30), 178 (100).

### Example 4:

### 1-(5-fluorobenzo[b]thiophen-3-yl)-3-[4-(indol-4-yl)piperazin-1-yl]propan-1-one

M.p.: 76-78°C

IR (KBr) (cm⁻¹): 3397 (m, NH); 1661 (s, C=O).

¹H-NMR (DMSO-d₆, 200 MHz) δ (ppm): 2.69 (b.s., 4H, N¹(CH₂)₂); 2.83 (t, 2H, COCH₂C*H*_{*2*}); 3.10 (b.s., 4H, N⁴(CH₂)₂); 3.31 (t, 2H, COC*H*_{*2*}CH₂); 6.41 (t, 2H, H₃'+H₅'); 6.97 (q, 2H, H₆'+H₇'); 7.33 (c, 1H, H₂'); 7.80 (d, 1H, H₆, J₆₇= 8.00); 8.12 (c, 1H, H₇), 8.30 (d, 1H, H₄, J_{F4}= 10.7); 9.13 (s, 1H, H₂); 10.98 (s, 1H, NH).

MS-DIP (70 eV) m/z (% Abundance) : 213 (7), 206 (50), 201 (49), 179 (100), 159 (88).

### Example 5:

### 1-(5-fluorobenzo[b]thiophen-3-yl)-3-[4-(6-propylendioxyphenyl)piperazin-1-yl]propan-1-one

IR (KBr) (cm⁻¹): 1670 (s, C=O).

¹H-NMR (CDCl₃, 200 MHz) δ (ppm) : 2.20 (q, 2H, CH₂); 2.73 (t, 4H, N¹(CH₂)₂); 2.95 (t, 2H, COCH₂C*H*_{*2*}); 3.09 (t, 4H, N⁴(CH₂)₂); 3.25 (t, 2H, COC*H*_{*2*}); 4.25 (q, 4H, O-(CH₂)₂-O), 6.63 (t, 2H, H₉+H₇), 6.84 (t, 1H, H₈), 7.20 (dd, 1H, H₆, J₆₇= 8.44, J₄₆= 2.5); 7.79 (c, 1H, H₇); 8.41 (s, 1H, H₂); 8.48 (dd, 1H, H₄ J_{F4}= 10.76).

### Example 6:

### 1-(5-fluorobenzo[b]thiophen-3-yl)-3-[4-(4-methylendioxyphenyl)piperazin-1-yl]propan-1-one

M.p.: 84-86°C

IR (KBr) (cm⁻¹): 1667 (s, C=O).

¹H-NMR (DMSO-d₆, 200 MHz) δ (ppm): 2.68 (t, 4H, N¹(CH₂)₂); 2.92 (t, 2H, COCH₂C*H*_{*2*}); 3.19 (t, 6H, COC*H*_{*2*}+N⁴(CH₂)₂); 5.91 (s, 2H, O-CH₂-O), 6.46 (c, 2H, H₅+H₇), 6.75 (t, 1H, H₆), 7.20 (ddd, 1H, H₆, J₆₇= 8.44, J₄₆= 2.5); 7.77 (c, 1H, H₇); 8.37 (s, 1H, H₂); 8.46 (dd, 1H, H₄ J_{F4}= 10.76).

### Example 7:

### 1-(benzo[b]thiophen-3-yl)-3-[4-(quinol-8-yl)piperazin-1-yl]propan-1-one dihydrochloride

Starting with 1.5 g (6.7 mmol) of the (benzo[b]thiophen-3-yl)-3-chloropropan-1-one and 1.4 g (6.7 mmol) of 1-(8-quinolil)-piperazine and following the procedure described in Example 1, 1.3 g of the product is made as a free base (yield 50%).

The product so obtained is dissolved en acetone and is precipitated as dihydrochloride by addition of HCl_{(c)}.

M.p.: 150-152°C

IR (KBr) (cm⁻¹): 1658 (s, C=O).

¹H-NMR (DMSO-d₆, 200 MHz) δ (ppm): 3.38 (b.s., 12H, CH₂-s); 7.24 (t, 1H, H₃'); 7.41-7.63 (m, 4H, H₄'+H₅'+ H₆'+ H₇'); 8.35-8.55 (m, 2H, H₅+H₆); 8.68 (d, 1H, H₄, J₄₅= 7.15); 8.70 (d, 1H, H₇, J₆₇= 8.7); 8.71 (dd, 1H, H₂'); 9.15 (s, 1H, H₂); 9.35 (s, 2H, HCl).

MS-DIP (70 eV) m/z (% Abundance): 401 (M⁺, 1), 213 (15) , 188 (53), 161 (100).

Following the procedure described in Example 7 and replacing the 1-(8-quinolil)-piperazine with the corresponding piperazine, the following products are made:

### Example 8:

### 1-(benzo[b]thiophen-3-yl)-3-[4-(quinaldin-8-yl)piperazin-1-yl]propan-1-one dihydrochloride

M.p.: 199-201°C

IR (KBr) (cm⁻¹): 1663 (s, C=O).

¹H-NMR (DMSO-d₆, 200 MHz) δ (ppm) : 2.86 (s, 3H, CH₃); 3.28-3.96 (m, 12H, CH₂-s); 7.41-7.73 (m, 6H, H₃'+ H₅'+ H₅+ H₆+H₆'+H₇'); 8.11 (c, 1H, H₄'); 8.50 (d, 1H, H₄, J₄₅= 8.04); 8.61 (t, 1H, H₇); 9.15 (s, 1H, H₂); 11.39 (b.s., 2H, HCl).

MS-DIP (70 eV) m/z (% Abundance): 227 (29), 188 (44), 171 (95), 161 (78), 143 (100).

### Example 9:

### 1-(benzo[b]thiophen-3-yl)-3-[4-(2,3-dihydro-1,4-benzodioxyn-5-yl)piperazin-1-yl]propan-1-one dihydrochloride

M.p.: 183-185°C
IR (KBr) (cm⁻¹): 1664 (s, C=O).
¹H-NMR (DMSO-d₆, 200 MHz) δ (ppm): 3.11 (t, 6H, N¹(CH₂)₃); 3.55 (t, 4H, N⁴(CH₂)₂) 3.76 (d, 2H, COCH₂); 4.28 (d, 4H, O(CH₂)₂); 6.53 (d, 2H, H₆'+H₈'); 6.76 (t, 1H, H₇'); 7.43-7.57 (m, 2H, H₅+H₆); 8.10 (dd, 1H, H₄, J₄₅= 8.3, J₄₆= 1.4); 8.61 (dd, 1H, H₇, J₆₇= 8.90, J₅₇= 1.5); 9.13 (s, 1H, H₂); 11.38 (b.s., 2H, HCl).

MS-DIP (70 eV) m/z (% Abundance): 220 (34), 188 (32), 178 (100), 161 (64), 149 (5).

### Example 10:

### 1-(benzo[b]thiophen-3-yl)-3-[4-(indol-4-yl)piperazin-1-yl]propan-1-one

M.p.: 83-85°C

IR (KBr) (cm⁻¹): 3397 (m, NH); 1661 (s, C=O).

¹H-NMR (DMSO-d₆, 200 MHz) δ (ppm): 2.69 (b.s., 4H, N¹(CH₂)₂); 2.83 (t, 2H, COCH₂C*H*_{*2*}); 3.10 (b.s., 4H, N⁴(CH₂)₂); 3.31 (t, 2H, COC*H*_{*2*}CH₂); 6.41 (t, 2H, H₃'+H₅'); 6.91-7.04 (m, 2H, H₅+H₆); 7.24 (t, 1H, H₂'); 7.43-7.56 (m, 2H, H₆'+H₇'); 8.10 (dd, 1H, H₄, J₄₅= 8.3, J₄₆= 1.7); 8.65 (d, 1H, H₇, J₇₆= 8.1); 9.06 (s, 1H, H₂); 11.03 (s, 1H, NH).

MS-DIP (70 eV) m/z (% Abundance): 389 (M⁺, 1), 201 (51), 188 (48), 161 (90), 159 (100), 144 (18).

### Example 11:

### 1-(benzo[b]thiophen-3-yl)-3-[4-(quinolin-5-yl)piperazin-1-yl]propan-1-one

¹H-NMR (CDCl₃, 200 MHz) δ (ppm): 2.92 (b.s., 4H, piperazine); 3.04 (t, 2H); 3.14 (b.s., 4H, piperazine); 3.30 (t.2H); 7.12 (d, 1H); 7.38-7.64 (m, 4H); 7.80-7.92 (m, 2H); 8.37 (s, 1H); 8.52 (d, 1H); 8.79 (d, 1H); 8.90 (d, 1H).

### Example 12:

### 1-(benzo[b]thiophen-3-yl)-3-[4-(2,3-dihydro-benzo[1,4]-dioxyn-6-yl)piperazin-1-yl]propan-1-one

¹H-NMR (CDCl₃, 200 MHz) δ (ppm): 2.68 (b.s., 4H, piperazine); 2.94 (t, 2H, CH₂); 3.10 (b.s., 4H, piperazine); 3.24 (t, 2H, CH₂); 4.22 (b.s., 4H, benzodioxane); 6.42-6.46 (m, 2H); 6.79 (d, 1H); 7.39-7.56 (m, 2H); 7.86 (d, 1H); 8.34 (s, 1H); 8.78 (d, 1H).

### Example 13:

### 1-(benzo[b]thiophen-3-yl)-3-[4-(3,4-dihydro-2H-benzo[b]-[1,4]dioxepin-6-yl)piperazin-1-yl]propan-1-one

¹H-NMR (CDCl₃, 200 MHz) δ (ppm): 2.10 (t, 2H, CH₂); 2.78 (b.s., 4H, piperazine); 2.98 (t, 2H, CH₂); 3.08 (b.s., 4H, piperazine); 3.24 (t, 2H, CH₂), 4.20-4.34 (m, 4H, benzodioxepine); 6.58-6.70 (m, 2H); 6.82 (t, 1H); 7.38-7.56 (m, 2H); 7.84 (d, 1H); 8.37 (s, 1H); 8.79 (d, 1H).

### Example 14:

### 1-(benzo[b]thiophen-3-yl)-3-[4-(quinol-2-yl)piperazin-1-yl]propan-1-one

M.p.: 92-96°C

IR (KBr) (cm⁻¹): 1664 (s, C=O).

¹H-NMR (CDCl_{3.2}00 MHz) δ (ppm) : 2.66 (t, 4H, N⁴(CH₂)₂); 2.94 (t, 2H, N¹(CH₂)); 3.25 (t, 2H, CO-CH₂); 3.76 (t, 4H, N¹(CH₂)₂); 6.96 (d, 1H, H₃', J = 7.9 Hz); 7.21-7.25 (m, 1H, H₆'); 7.41-7.56 (m, 4H, H₅ + H₆ + H₅' + H₇'); 7.68 (d, 1H, H₄'); 7.85-7.90 (m, 2H, H₇ + H₈'); 8.33 (s, 1H, H₂); 8.74 (d, 1H, H₄).

### Example 15:

### 1-(benzo[b]thiophen-3-yl)-3-[4-(quinol-4-yl)piperazin-1-yl]propan-1-one

M.p.: 134-136°C

IR (KBr) (cm⁻¹): 1615 (s, C=O).

¹H-NMR (CDCl₃,.₂00 MHz) δ (ppm): 2.81-2.85 (m, 4H, N⁴(CH₂)₂); 3.02 (t, 2H, CO-(CH₂)); 3.24-3.32 (m, 6H, N¹(CH₂)₃); 6.84 (d, 1H, H₃', J = 4.9 Hz); 7.43-7.51 (m, 3H, H₅ + H₆ + H₆'); 7.65 (t, 1H, , H₇');7.85 (t, 1H, H₇); 7.99-8.06 (m, 2H, H₅'+H₈'); 8.35 (s, 1H, H₂); 8.71-8.79 (m, 2H, H₂'+H₄).

### Example 16:

### 1-(benzo[b]thiophen-3-yl)-3-[4-(quinol-3-yl)piperazin-1-yl]propan-1-one

M.p.: 112-114°C

IR (KBr) (cm⁻¹): 1665 (s, C=O).

¹H-NMR (CDCl₃.200 MHz) δ (ppm) : 2.75 (t, 4H, N⁴(CH₂)₂); 2.96 (t, 2H, CO-CH₂)); 3.21-3.36 (m, 6H, N¹(CH₂)₃); 7.31 (d, 1H, H₄', J= 1.9 Hz); 7.37-7.53 (m, 4H, H₅ + H₆ + H₆' + H₈'); 7.65 (dd, 1H, H₅', J = 6 Hz; J' = 2 Hz); 7.86 (d, 1H, H₇'); 7.97 (d, 1H, H₇, J = 8 Hz); 8, 32 (s, 1H, H₂); 8.74 (s, 1H, H₂'); 8.78 (d, 1H, H₄).

### Example 17:

### 1-(benzo[b]thiophen-3-yl)-3-[4-quinolin-6-yl)piperazin-1-yl]propan-1-one

¹H-NMR (CDCl₃, 200 MHz) δ (ppm): 2.78 (b.s., 4H, piperazine); 2.98 (t, 2H, CH₂); 3.22-3.40 (m, 6H, 4H piperazine + CH₂); 7.01 (d, 1H); 7.24-7.56 (m, 4H); 7.82-8.04 (m, 3H); 8.38 (s, 1H); 8.70-8.80 (m, 2H).

### Example 18:

### 6-[4-(3-benzo[b]thiophen-3-yl-oxo-propil)-piperazin-1-yl]-1H-indol-2-carboxylic acid, ethyl ester

¹H-NMR (CDCl₃, 200 MHz) δ (ppm): 1.42 (t, 3H, CH₃); 2.80 (b.s., 4H, piperazine); 3.00 (t, 2H, CH₂); 3.22-3.36 (m, 6H, 4H piperazine + CH₂); 4.41 (q, 2H, CH₂); 6.58 (d, 1H); 7.02 (d, 1H); 7.08-7.14 (m, 2H); 7.39-7.56 (m, 2H); 7.86 (d, 1H); 8.37 (s, 1H); 8.79 (d, 1H); 9.02 (b.s., 1H, NH).

### Example 19:

### 4-[4-(3-benzo[b]thiophen-3-yl-oxo-propil)-piperazin-1-yl]-1H-indol-2-carboxylic acid, ethyl ester

¹H-NMR (CDCl₃, 200 MHz) δ (ppm): 1.39 (t, 3H, CH₃); 2.70 (b.s., 4H, piperazine); 2.97 (t, 2H, CH₂); 3.18-3.34 (m, 6H, 4H piperazine + CH₂); 4.38 (q, 2H, CH₂); 6.79 (s, 1H); 6.90 (dd, 1H); 7.12 (d, 1H); 7.38-7.56 (m, 3H); 7.86 (d, 1H); 8.36 (s, 1H); 8.79 (d, 1H); 8.81 (b.s., 1H, NH).

### Example 20:

### 1-(5-methylbenzo[b]thiophen-3-yl)-3-[4-(2,3-dihydro-1,4-benzodioxyn-5-yl)piperazin-1-yl]propan-1-one

### 1. (5-methylbenzo[b]thiophen-3-yl)-3-chloropropan-1-one

Onto 900 mg of aluminium trichloride dissolved in 36 ml dry chloroform in nitrogen atmosphere is added dropwise a solution of 1 g (6.75 mmol) of 5-methylbenzo[b]thiophene and 0.6 ml (6.75 mmol) 2-chloropropionyl chloride dissolved in 70 ml of dry chloroform. After 24 hours of reaction, 50 ml of HCl 1.5 N is added, left under agitation for 2 hours and decanted, and the organic phase phase is subsequently washed with a dilute solution of sodium bicarbonate, with H₂O and with a saturated solution of sodium chloride. It is dried with sodium sulphate, the solvent is eliminated and it is purified with a silica column using hexane/toluene (90:10) as mobile phase. (Yield: 80%).

M.p.: 100-102°C

IR (KBr) (cm⁻¹): 1675 (s, C=O).

¹H-NMR (CDCl₃, 200 MHz) δ (ppm): 2.61 (s, 3H, CH₃); 3.46 (c, 2H, ClCH₂); 3.46 (c, 2H, COCH₂); 7.26 (t, 1H, H₆); 7.75 (d, 1H, H₇); 8.29 (s, 1H, H₂); 8.59 (s, 1H, H₄).

### 2. 1-(5-methylbenzo[b]thiophen-3-yl)-3-[4-(2,3-dihydro-1,4-benzodioxyn-5-yl)piperazin-1-yl]propan-1-one

Following the procedure described in Example 1 and starting with (5-methylbenzo[b]thiophen-3-yl)-3-chloropropan-2-one and 1-(2,3-dihydro-benzo[1,4]dioxyn-5-yl)piperazine, the product of the title is made.

M.p.: 132-133°C

IR (KBr) (cm⁻¹): 1673 (s, C=O).

¹H-NMR (CDCl₃, 200 MHz) δ (ppm): 2.46 (s, 3H, CH₃); 2.70 (b.s., 4H, N¹(CH₂)₂); 2.93 (d, 2H, COCH₂C*H*_{*2*}); 3.06 (b.s., 4H, N⁴(CH₂)₂) 3.18 (d, 2H, COC*H*_{*2*}); 4.20-4.28 (m, 4H, O(CH₂)₂); 6.47-6.57 (m, 2H, H₆'+H₈'); 6.73 (t, 1H, H₇'); 7.21 (s, 1H, H₆); 7.69 (d, 1H, H₇); 8.26 (s, 1H, H₂); 8.54 (s, 1H, H₄).

### Example 21:

### 1-(5-nitrobenzo[b]thiophen-3-yl)-3-[4-(2,3-dihydro-1,4-benzodioxyn-5-yl)piperazin-1-yl]propan-1-one

### 1. (5-nitrobenzo[b]thiophen-3-yl)-3-chloropropan-1-one

Onto 650 mg of AlCl₃ dissolved in 20 ml dry chloroform in nitrogen atmosphere is added dropwise a solution of 1 g (5.58 x 10⁻³ moles) of 5-nitrobenzo[b]thiophene and 0.65 ml (6.64 x 10⁻³ moles) of 2-chloropropionyl chloride dissolved in 40 ml of dry chloroform. It is allowed to react for 24 hours at ambient temperature and the same amounts of the AlCl₃ and the acid chloride are added. After 48 hours of reaction 100 ml of HCl 1.5 N is added and it is decanted, and the organic phase is washed subsequently with a dilute solution of NaHCO₃, with H₂O and with a saturated solution of NaCl. It is dried with Na₂SO₄, the solvent is eliminated and it is purified in a silica column using hexane/toluene (25:75) as mobile phase. Yield: 30%.

M.p.: 128 °C

IR (KBr) (cm⁻¹): 1670 (s, C=O); 1510, 1335 (s, NO₂).

¹H-NMR (CDCl₃, 200 MHz) δ (ppm): 3.51 (t, 2H, C*H*_{*2*}C=O); 3.98 (t, 2H, C*H*_{*2*}Cl); 7.99 (d, 1H, H₇), 8.29 (dd, 1H, H₆); 8.49 (s, 1H, H₂); 9.64 (d, 1H, H₄).

MS-DIP (70 eV) m/z (% Abundance): 269 (M⁺; 17); 206 (100).

### 2. 1-(5-nitrobenzo[b]thiophen-3-yl)-3-[4-(2,3-dihydro-1,4-benzodioxyn-5-yl)piperazin-1-yl]propan-1-one

Following the procedure described in Example 1 and starting from (5-nitrobenzo[b]thiophen-3-yl)-3-chloropropan-1-one and 1-(2,3-dihydro-benzo[1,4]dioxyn-5-yl)piperazine the product of the title is made.

M.p.: 168-171°C

¹H-NMR (CDCl₃, 200 MHz) δ (ppm) : 2.71 (t, 4H, N¹(CH₂)₂); 2.93 (t, 2H, COCH₂C*H*_{*2*}); 3.06 (t, 4H, N⁴(CH₂)₂) 3.23 (t, 2H, COC*H*_{*2*}); 4.20 (d, 4H, O(CH₂)₂); 6.47-6.57 (d, 2H, H₆'+H₈'); 6.73 (t, 1H, H₇'); 7.33 (d, 1H, H₇); 8.22 (dd, 1H, H₆, J₆₇= 9.05, J₄₆= 2.01); 8.18 8.46 (s, 1H, H₂), 9.59 (d, 1H, H₄, J₄₆= 1.94);

### Example 22:

### 3-[4-(1H-indol-4-yl)piperazin-1-yl]-1-(5-nitrobenzo[b]-thiophen-3-yl)propan-1-one

Following the procedure of the previous example and using the 4-piperazin-1-yl-1H-indol as piperazine, the product of the title is made.

¹H-NMR (CDCl₃, 200 MHz) δ (ppm): 2.80 (b.s., 4H, piperazine); 3.01 (t, 2H, CH₂); 3.22-3.36 (m, 6H, 4H piperazine + CH₂); 6.54-6.60 (m, 2H); 7.02-7.16 (m, 3H); 7.88 (d, 1H); 8.22-8.32 (m, 2H); 8.50 (s, 1H); 9.62 (d, 1H).

### Example 23:

### 1-(5-acetoxybenzo[b]thiophen-3-yl)-3-[4-(2,3-dihydro-1,4-benzodioxyn-5-yl)piperazin-1-yl]propan-1-one

### 1. 5-acetoxybenzo[b]thiophene

Onto 450 mg of 5-hydroxybenzo[b]thiophene (3 x 10⁻³ moles) in 5 ml of benzene is added 1 ml of Ac₂O in three additions while in reflux. After 2 hours (followed by T.L.C. with toluene) the solvent is eliminated and the solid is washed with cold EtOH. Yield: 85%.

M.p.: 68°C

IR (KBr) (cm⁻¹): 1735 (s, C=O)

¹H-NMR (CDCl₃, 200 MHz) δ (ppm): 2.27 (s, 3H, CH₃); 7.00 (dd, 1H, H₆); 7.23 (d; 1H, H₂); 7.43 (d, 1H, H₃); 7.47 (d; 1H, H₄); 7.78 (d, 1H, H₇).

MS-DIP (70 eV) m/z (% Abundance): 192 (M⁺.17); 150 (100).

### 2. (5-acetoxybenzo[b]thiophen-3-yl)-3-chloropropan-1-one

Onto 1.04 g of aluminium trichloride dissolved in 25 ml dry chloroform in nitrogen atmosphere is added dropwise a solution of 1.5 g (7.81 mmol) of 5-acetoxybenzo[b]thiophene and 1 ml (7.81 mmol) of 2-chloropropionyl chloride dissolved in 50 ml of dry chloroform. After 24 hours of reaction, 0.5 ml of sulphuric acid and 7.5 ml of of water are added dropwise. It is left under agitation and is extracted with dichloromethane. It is dried with sodium sulphate, the solvent is eliminated and it is purified in a silica column using hexane/toluene (25:75) as mobile phase. (Yield: 43%).

¹H-NMR (CDCl_{3.2}00 MHz) δ (ppm): 2.33 (s, 3H, CH₃); 3.08 (t, 2H, ClCH₂); 3.88 (t, 2H, COCH₂); 7.17 (dd, 1H, H₇, J₆₇= 8.64); 7.83 (d, 1H, H₆, J₆₇= 8.61); 8.34 (s, 1H, H₂); 8.48 (d, 1H, H₄, J₄₆= 2.2).

### 3. 1-(5-acetoxybenzo[b]thiophen-3-yl)-3-[4-(2,3-dihydro-1,4-benzodioxin-5-yl)piperazin-1-yl]propan-1-one

Following the procedure described in Example 1, starting with (5-acetoxybenzo[b]thiophen-3-yl)-3-chloropropan-1-one and 1-(2,3-dihydro-benzo[1,4]dioxyn-5-yl)piperazine, the product of the title is made.

M.p.: 106-109°C

IR (KBr) (cm⁻¹): 1261 (s, Ar-O); 1660 (s, C=O); 1757 (s, O-C=O).

¹H-NMR (CDCl₃, 200 MHz) δ (ppm): 2.30 (s, 3H, CH₃); 2.72 (b.s., 4H, CH₂); 2.92 (t, 2H, CH₂); 3.07 (b.s., 4H, CH₂); 3.20 (t, 2H, CH₂); 4.24 (d, 4H, OCH₂); 6.48-6.57 (m, 2H, H_{5'}+ H_{7'}); 6.74 (t, 1H, H_{6'}); 7.14 (d, 1H, H₆); 7.80 (d, 1H, H₇); 8.33 (s, 1H, H₂); 8.46 (s, 1H, H₄).

### Example 24:

### 1-(benzo[b]thiophen-3-yl)-3-[4-(indol-4-yl)piperazin-1-yl]etan-1-one

Starting with 1-benzo[b]thiophen-3-yl-2-bromo-etanone and 4-piperazin-1-yl-1H-indol and following the procedure described in Example 1, the product of the title is made.

M.p. (as hydrochloride): 297-300°C

IR (KBr) (cm⁻¹): 3360(m, NH), 1672 (s, C=O).

¹H-NMR (CDCl₃, 200 MHz) δ (ppm): 2.69 (t, 4H, N¹(CH₂)₂); 3.44 (t, 4H, N⁴(CH₂)₂) 3.85 (s, 2H, COC*H*_{*2*}); 6.53-6.62 (m, 2H, H₃'+H₅'); 7.08-7.16 (m, 2H, H₆'+H₇'); 7.44-7.50 (m, 2H, H₅+H₆); 7.87 (d, 1H, H₂'); 8.17 (b.s., 1H, H₄); 8.78 (d, 1H, H₇+H₂)

### Example 25:

### 1-Benzo[b]thiophen-3-yl-3-[4-(2,3-dihydro-benzofuran-7-yl)-piperazin-1-yl]-propan-1-one

Starting with 1-benzo[b]thiophen-3-yl-3chloro-1-propanone and 1-(7-benzofuranyl)piperazine and following the procedure described in Example 1, the product of the title is made.

¹H-NMR (CDCl₃, 200 MHz, δ ppm) : 8.78 (d, 1H); 8.35 (s, 1H); 7.84 (d, 1H); 7.54-7.38 (m, 2H); 6.84-6.68 (m, 3H); 4.60 (t, 2H, OCH2); 3.36-3.10 (m, 8H); 2.98 (t, 2H); 2.80-2.70 (m, 4H)

IR: 2820 cm⁻¹, 1670 cm⁻¹, 1490 cm⁻¹, 1460 cm⁻¹

### Example 26:

### 1-(Benzo[b]thiophen-3-yl)-3-(4-benzo[b]thiophen-5-yl-piperazin-1-yl)propan-1-one

Starting with 1-benzo[b]thiophen-3-yl-3chloro-1-propanone and of 1-(5-benzothiophenil)piperazine and following the procedure described in Example 1, the product of the title is made.

¹H-NMR (CDCl₃, 200 MHz) δ (ppm): 2.78 (b.s., 4H, CH₂); 3.00 (t, 2H, CH₂) 3.26 (b.s., 4H, CH₂); 7.07 (d, 1H, H₆'); 7.21-7.30 (m, 4H, H₂'+H₃'+H₆'+H₇'); 7.68-7.75 (m, 2H, H₅+H₆); 7.70 (d, 1H, H₄); 8.35 (s, 1H, H₂); 8.77 (d, 1H, H₄)

IR (KBr) (cm⁻¹): 1656 (s, C=O).

### Method B

### General procedure

To 4 mmol of the ketones of examples 1-26 dissolved in 50 ml of methanol cooled to 0°C is added dropwise a solution of 4.2 mmol of sodium borohydride dissolved in 1.4 ml of water and 0.14 ml of NaOH 2N.

It is heated under reflux for 6-8 h and left at ambient temperature overnight.

0.28 ml of HCl 1N is added and in cases in which the product precipitates out it is filtered.

If it does not precipitate out, it is concentrated to dryness and the residue is divided between CH₂Cl₂/H₂O. The organic phase is dried in 2-propanol for 10-15 minutes filtering the precipitated solid or is chromatographed onto silica gel eluting with CH₂Cl₂/MeOH 90/10.

In order to prepare the corresponding hydrochloride, once the product has been isolated and precipitated, it is dissolved in a mixture, is agitated and 1 equivalent of HCl(c) is added. The mixture is agitated until the hydrochloride precipitates, which precipitate is then filtered and dried.

Following this general procedure, the following products have been obtained:

### Example 27:

### 1-(5-fluorobenzo[b]thiophen-3-yl)-3-[4-(quinol-8-yl) piperazin-1-yl]propan-1-ol

The product of the title is made following the general procedure, starting with the ketone synthesised in Example 1.

M.p.: 89-91°C

IR (KBr) (cm⁻¹): 3102 (w, OH).

¹H-NMR (DMSO-d₆ .₂00 MHz) δ (ppm): 2.52 (t, 2H, C*H*_{*2*}CHOH); 3.37 (b.s., 12H, CH₂-s); 5.03 (t, 1H, CHOH), 5.66 (b.s., 1H, OH); 7.09 (t, 2H, H₆+H₇); 7.19 (t, 1H, H₃'); 7.23-7.28 (m, 3H, H₅' + H₆'+H₇'); 7.68 (s, 1H, H₂); 7.97 (c, 1H, H₄'); 8.26 (d, 1H, H₄, J= 7.40); 8.82 (d, 1H, H₂').

MS-DIP (70 eV) m/z (% Abundance): 421 (M⁺, 11), 278 (2), 264 (2), 157 (100), 129 (49).

### Example 28:

### 1-(5-fluorobenzo[b]thiophen-3-yl)-3-[4-(2,3-dihydro-1,4-benzodioxyn-5-yl)piperazin-1-yl]propan-1-ol

The product of the title is made following the general procedure and starting with the ketone synthesised in Example 3.

M.p.: 147-150°C

IR (KBr) (cm⁻¹): 3360 (w, OH).

¹H-NMR (CDCl_{3 .2}00 MHz) δ (ppm): 2.05 (q, 2H, CHOHC*H*_{*2*}); 2.64 (m, 6H, N¹(CH₂)₃); 3.13 (b.s., 4H, N⁴(CH₂)₂); 4.24 (d, 2H, 2He, J= 5.6); 4.30 (d, 2H, Ha, J= 13.6); 5.25 (t, 1H, CHOH); 6.55 (c, 2H, H₆'+H₈'); 6.77 (t, 1H, H₇'); 6.98-7.13 (m, 2H, H₆+H₄);7.44 (s, 1H, H₂); 7.75 (c, 1H, H₇).

MS-DIP (70 eV) m/z (% Abundance): 428 (M⁺, 13), 233 (94), 178 (100), 164 (34), 133 (44).

### Example 29:

### 1-(5-fluorobenzo[b]thiophen-3-yl)-3-[4-(indol-4-yl)piperazina-1-yl]propan-1-ol

The product of the title is made following the general procedure and starting with the ketone synthesised in Example 4.

M.p.: 170-173°C

IR (KBr) (cm⁻¹): 3414 (w, OH).

¹H-NMR (DMSO-d₆, 200 MHz) δ (ppm): 1.99 (d, 2H, CHOHC*H*_{*2*}); 2.55 (d, 6H, N¹(CH₂)₃); 2.55 (b.s., 4H, N⁴(CH₂)₂); 5.02 (s, 1H, CHOH); 5.64 (b.s., 1H, OH); 6.41 (t, 2H, H₃'+H₅'); 6.98 (t, 3H, H₂'+H₆'+H₇'); 7.29 (s, 1H, H₂); 7.69 (d, 1H, H₄, J_{F7}= 4.92); 7.76 (d, 1H, H₆, J₄₆= 2.48); 8.01 (t, 1H, H₇).

MS-DIP (70 eV) m/z (% Abundance): 409 (M⁺), 228 (25), 214 (100), 157 (16).

### Example 30:

### 1-(5-fluorobenzo[b]thiophen-3-yl)-3-[4-(quinaldin-8-yl)piperazin-1-yl]propan-1-ol

The product of the title is made following the general procedure and starting with the ketone synthesised in Example 2.

M.p.: 148-150°C

IR (KBr) (cm⁻¹): 3414 (w, OH).

¹H-NMR (CDCl₃, 200 MHz) δ (ppm): 2.37 (t, 2H, CHOHC*H*_{*2*}); 2.94 (s, 3H, CH₃); 3.38 (b.s., 6H, N¹(CH₂)₃); 3.76 (t, 4H, N⁴(CH₂)₂); 5.11 (t, 1H, CHOH); 7.37-7.45 (m, 2H, H₆'+H₇'); 7.54-7.85 (m, 5H, H₆+H₇+H₅'+H₄'+H₂); 7.97-8.07 (m, 1H, H₄); 8.67 (d, 1H, H₃'); 12.21 (b.s., 1H, OH).

MS-DIP (70 eV) m/z (% Abundance): 435 (M⁺, 6), 278 (2), 197 (13), 184 (17), 171 (100), 158 (28).

### Example 31:

### 1-(5-fluorobenzo[b]thiophen-3-yl)-3-[4-(6-propylendioxyphenyl)piperazin-1-yl]propan-1-ol

The product of the title is made following the general procedure and starting with the ketone synthesised in Example 5.

M.p.: 125-127°C

IR (KBr) (cm⁻¹): 3414 (m, OH).

¹H-NMR (CDCl_{3.2}00 MHz) δ (ppm): 2.03 (q, 2H, CH₂); 2.17 (t, 2H, CHOHC*H*₂); 2.64-2.80 (m, 6H, N¹(CH₂)₃); 3.09 (b.s., 4H, N⁴(CH₂)₂); 4.23 (q, 4H, O-(CH₂)₂-O), 5.23 (t, 1H, C*H*OH); 6.55-6.66 (m, 2H, H₉+H₇), 6.81 (t, 1H, H₈), 7.06 (ddd, 1H, H₆, J₆₇= 8.44, J₄₆= 2.5); 7.42-7.48 (m, 2H, H₂+H₇); 7.73 (c, 1H, H₄).

### Exaimple 32:

### 1-(5-fluorobenzo[b]thiophen-3-yl)-3-[4-(4-methylendioxyphenyl)piperazin-1-yl]propan-1-ol

The product of the title is made following the general procedure and starting with the ketone synthesised in Example 6.

M.p.: 147-149°C

IR (KBr) (cm⁻¹): 3415 (m, OH)

¹H-NMR (CDCl₃, 200 MHz) δ (ppm): 2.00-2.09 (m, 2H, CHOHC*H*_{*2*}); 2.64-2.87 (m, 6H, N¹(CH₂)₃); 3.24 (t, 4H, N⁴(CH₂)₂); 5.24 (t, 1H, C*H*OH); 5.92 (s, 2H, O-CH₂-O), 6.47 (c, 2H, H₅+H₇), 6.77 (t, 1H, H₆), 7.09 (ddd, 1H, H₆, J₆₇= 8.44, J₄₆= 2.5); 7.44-7.50 (m, 2H, H₂+H₇); 7.75 (c, 1H, H₄).

### Example 33:

### 1-(benzo[b]thiophen-3-yl)-3-[4-(quinol-8-yl)piperazin-1-yl]propan-1-ol hydrochloride

The product of the title is made following the general procedure and starting with the ketone synthesised in Example 7.

M.p.: 169-170°C

IR (KBr) (cm⁻¹): 3414 (w, OH).

¹H-NMR (DMSO-d₆, 200 MHz) δ (ppm): 2.13 (t, 2H, C*H*_{*2*}CHOH); 3.39 (b.s., 12H, CH₂-s); 5.11 (t, 1H, CHOH), 7.40 (q, 2H, H₃'+H₄'); 7.58 (d, 1H, H₅', J= 7.34); 7.72 (t, 2H, H₆'+H₇'); 7.86 (t, 2H, H₅+H₆); 8.04 (q, 2H, H₇+H₄); 9.08 (d, 1H, H₂'); 9.33 (s, 1H, H₂); 10.23 (b.s., 1H, HCl); 11.19 (b.s., 1H, OH).

MS-DIP (70 eV) m/z (% Abundance): 403 (M⁺, 13), 260 (2), 246 (4), 170 (34), 157 (100), 129 (58).

### Example 34:

### 1-(benzo[b]thiophen-3-yl)-3-[4-(2,3-dihydro-1,4-benzodioxyn-5-yl)piperazin-1-yl]propan-1-ol

The product of the title is made following the general procedure and starting with the ketone synthesised in Example 9.

M.p.: 151-153°C

IR (KBr) (cm⁻¹): 3414 (w, OH).

¹H-NMR (CDCl₃, 200 MHz) δ (ppm) : 2.08 (b.s., 2H, CHOHC*H*_{*2*}); 2.73 (b.s., 6H, N¹(CH₂)₃); 3.12 (b.s., 4H, N⁴(CH₂)₂); 4.26 (d, 4H, O-CH₂-CH₂-O); 5.33 (b.s., 1H, CHOH); 6.54 (t, 2H, H₆'+H₈'); 6.77 (t, 1H, H₇'); 7.33 (t, 2H, H₅+H₆); 7.41 (s, 1H, H₂); 7.78 (d, 1H, H₄+H₇, J= 7.60).

MS-DIP (70 eV) m/z (% Abundance): 410 (M⁺, 4), 247 (16), 233 (74), 178 (100).

### Example 35:

### 1-(benzo[b]thiophen-3-yl)-3-[4-(indol-4-yl)piperazin-1-yl]propan-1-ol hydrochloride

The product of the title is made following the general procedure and starting with the ketone synthesised in Example 10.

M.p.: >300°C

IR (KBr) (cm⁻¹): 3396 (w, OH).

¹H-NMR (DMSO-d₆, 200 MHz) δ (ppm): 2.00 (t, 2H, CHOHC*H*_{*2*})*;* 2.55 (t, 6H, N¹(CH₂)₃); 3.28 (b.s., 4H, N⁴(CH₂)₂); 5.33 (d, 1H, CHOH); 5.62 (b.s., 1H, OH); 6.42 (t, 2H, H₃'+H₅'); 6.99 (q, 2H, H₆+H₅); 7.23-7.43 (m, 3H, H₂'+H₆'+H₇'); 7.57 (s, 1H, H₂); 7.96 (q, 2H, H₄+H₇); 10.42 (b.s., 1H, HCl).

MS-DIP (70 eV) m/z (% Abundance): 391 (M⁺, 34), 228 (18), 214 (100), 159 (5).

### Example 36:

### 1-(benzo[b]thiophen-3-yl)-3-[4-(quinaldin-8-yl)piperazin-1-yl)propan-1-ol hydrochloride

The product of the title is made following the general procedure and starting with the ketone synthesised in Example 8.

M.p.: 138-142°C

IR (KBr) (cm⁻¹): 3358 (w, OH).

¹H-NMR (DMSO-d₆, 200 MHz) δ (ppm): 2.37 (t, 2H, CHOHC*H*_{*2*}); 2.94 (s, 3H, CH₃); 3.38 (b.s., 6H, N¹(CH₂)₃); 3.76 (t, 4H, N⁴(CH₂)₂); 5.11 (t, 1H, CHOH); 7.37-7.45 (m, 2H, H₆'+H₇'); 7.54-7.85 (m, 5H, H₆+H₅+H₅'+H₄'+H₂); 7.97-8.07 (m, 2H, H₄+H₇); 8.67 (d, 1H, H₃', *J*= 8.22); 10.48 (b.s., 1H, HCl); 12.21 (b.s., 1H, OH).

MS-DIP (70 eV) m/z (% Abundance): 241 (3), 171 (100), 143 (55).

### Example 37:

### 1-(benzo[b]thiophen-3-yl)-3-[4-(quinol-5-yl)piperazin-1-yl]propan-1-ol

The product of the title is made following the general procedure and starting with the ketone synthesised in Example 11.

M.p.: 127-129°C

IR (KBr) (cm⁻¹): 3358 (w, OH).

¹H-NMR (DMSO-d₆, 200 MHz) δ (ppm): 2.00 (d, 2H, C*H*_{*2*}CHOH); 2.60 (t, 6H, N¹(CH₂)₃); 3.02 (s, 4H, N⁴(CH₂)₂); 5.08 (t, 1H, C*H*OH), 5.62 (b.s., 1H, OH); 7.15 (d, 1H, H₄'); 7.21-7.43 (m, 3H, H₅+H₆+H₇'); 7.46-4.88 (m, 3H, H₂'+H₃'+H₆'); 7.89-7.98 (m, 2H, H₇+H₄); 8.44 (d, 1H, H₈', *J*=8.39); 8.87 (s, 1H, H₂).

MS-DIP (70 eV) m/z (% Abundance): 403 (M⁺, 11), 240 (8), 226 (100), 171 (41), 129 (4).

### Example 38:

### 1-(benzo[b]thiophen-3-yl)-3-[4-(indol-4-yl)piperazin-1-yl]ethan-1-ol

The product of the title is made following the general procedure and starting with the ketone synthesised in Example 24.

M.p.: 164-165°C

IR (KBr) (cm⁻¹): 3400 (w, OH).

¹H-NMR (CDCl₃, 200 MHz) δ (ppm): 2.71-2.84 (m, 4H, N¹(CH₂)₂); 3.02 (d, 2H, C*H*_{*2*}CHOH); 3.31 (s, 4H, N⁴(CH₂)₂) 5.23 (d, 1H, CHOH); 6.58 (t, 2H, H₃'+H₅'); 7.07-7.14 (m, 2H, H₅+H₆); 7.33 (t, 2H, H₂'+H₆'); 7.45 (s, 1H, H₂); 7.83 (d, 2H, H₇+H₄).

MS-DIP (70 eV) m/z (% Abundance): 403 (M⁺, 11) , 240 (8), 226 (100), 171 (41), 129 (4).

### Example 39:

### 1-(5-nitrobenzo[b]thiophen-3-yl)-3-[4-(2,3-dihydro-1,4-benzodioxyn-5-yl)piperazin-1-yl)propan-1-ol

The product of the title is made following the general procedure and starting with the ketone synthesised in Example 21.

M.p.: 105-107°C.

IR (KBr) (cm⁻¹): 3360 (m, OH).

¹H-NMR (CDCl₃, 200 MHz) δ (ppm): 2.05 (t, 2H, CHOHC*H*_{*2*}), 2.71-2.96 (m, 6H, N¹(CH₂)₃); 3.15 (b.s., 4H, N⁴(CH₂)₂); 4.25 (d, 4H, O(CH₂)₂); 5.37 (c, 1H, C*H*OH); 6.52-6.62 (d, 2H, H₆'+H₈'); 6.78 (t, 1H, H₇'); 7.59 (s, 1H, H₂); 7.94 (d, 1H, H₇); 8.18 (dd, 1H, H₆, *J*_{*67*}= 9.05, *J*_{*46*}= 2.01); 8.76 (d, 1H, H₄, *J*_{*46*}= 1.94)

### Example 40:

### 1-(5-methylbenzo[b]thiophen-3-yl)-3-[4-(2,3-dihydro-1,4-benzodioxyn-5-yl)piperazin-1-yl)propan-1-ol

The product of the title is made following the general procedure and starting with the ketone synthesised in Example 20.

M.p.: 144-147°C

IR (KBr) (cm⁻¹): 3414 (m, OH).

¹H-NMR (CDCl₃, 200 MHz) δ (ppm): 2.13 (t, 2H, C*H*_{*2*}CHOH); 2.5 (s, 3H, CH₃); 2.70-2.84 (m, 8H, piperazine); 3.18 (d, 2H, CHOHCH₂C*H*_{*2*}); 4.20-4.28 (m, 4H, O(CH₂)₂); 6.54-6.65 (m, 2H, H₆'+H₈'); 6.82 (t, 1H, H₇'); 7.19 (s, 1H, H₆); 7.43 (s, 1H, H₄); 7.60 (s, 1H, H₂); 7.76 (d, 1H, H₇).

### Example 41:

### 3-[4-(1H-indol-4-yl)piperazin-1-yl]-1-(5-nitrobenzo[b]-thiophen-3-yl)propan-1-ol

The product of the title is made following the general procedure and starting with the ketone synthesised in Example 22.

¹H-NMR (CDCl₃, 200 MHz) δ (ppm): 2.10 (m, 2H, CH₂); 2.54-2.78 (m, 6H, 4H piperazin + CH₂); 3.18 (b.s., 4H piperazin); 5.22 (t, 1H, 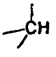 ); 5.90 (b.s., 1H, OH); 6.41 (s, 1H); 6.50 (d, 1H); 6.98-7.12 (m, 2H); 7.32 (s, 1H); 7.94 (s, 1H); 8.24 (d, 1H); 8.36 (d, 1H); 8.92 (s, 1H); 11.18 (b.s., 1H, NH)

### Example 42:

### 1-(benzo[b]thiophen-3-yl)-3-[4-(2,3-dihydrobenzo[1,4]dioxyn-6-yl)piperazin-1-yl]propan-1-ol

The product of the title is made following the general procedure and starting with the ketone synthesised in Example 12.

¹H-NMR (CDCl₃, 200 MHz) δ(ppm): 2.02-2.14 (m, 2H, CH₂); 2.60-2.84 (m, 6H, 4H piperazin + CH₂); 3.14 (b.s., 4H piperazin); 4.23 (b.s., 4H, OCH₂); 5.30-5.40 (m, 1H, CH); 6.42-6.50 (m, 2H); 6.70-6.82 (m, 2H); 7.24-7.40 (m, 2H); 7.42 (s, 1H); 7.76-7.90 (m, 2H); 11.20 (b.s., 1H, NH)

### Example 43:

### 1-(benzo[b]thiophen-3-yl)-3-[4-(3,4-dihydro-2H-benzo-[b][1,4]dioxepin-6-yl)piperazin-1-yl]propan-1-ol

The product of the title is made following the general procedure and starting with the ketone synthesised in Example 13.

¹H-NMR (CDCl₃, 200 MHz) δ(ppm): 2.04-2.16 (m, 2H, CH₂); 2.16-2.26 (m, 2H, CH₂); 2.64-2.90 (m, 6H, 4H piperazin + CH₂); 3.16 (b.s., 4H piperazin); 4.22-4.32 (m, 4H, 2 OCH₂); 5.34-5.42 (m, 1H, CH); 6.60-6.74 (m, 2H); 6.86 (t, 1H); 7.34-7.40 (m, 2H); 7.42 (s, 1H); 7.78-7.92 (m, 2H).

### Example 44:

### 1-(benzo[b]thiophen-3-yl)-3-[4-(quinol-2-yl)piperazin-1-yl]propan-1-ol

The product of the title is made following the general procedure and starting with the ketone synthesised in Example 14.

M.p. (as hydrochloride): 192-196°C

IR (KBr) (cm-1): 3149 (w, OH).

¹H-NMR (CDCl₃, 200 MHz) δ (ppm): 2.12 (t, 2H, C*H*_{*2*}CHOH); 2.64-2.76 (m, 6H, N¹(CH₂)₃); 3.82 (t, 4H, N⁴(CH₂)₂); 5.37 (t, 1H, C*H*OH), 6.98 (d, 1H, H₃, *J*=7.8 Hz); 7.24-7.92 (m, 10H, H aromatic).

### Example 45:

### 1-(benzo[b]thiophen-3-yl)-3-[4-(quinol-4-yl)piperazin-1-yl]propan-1-ol hydrochloride

The product of the title is made following the general procedure and starting with the ketone synthesised in Example 15.

M.p.: 174-176°C

IR (KBr) (cm-1): 3277 (w, OH).

¹H-NMR (DMSO-d₆ .200 MHZ) δ(ppm): 2.02 (t, 2H, C*H*_{*2*}CHOH); 2.50-2.68 (m, 6H, N¹(CH₂)₃); 3.19 (b.s., 4H, N⁴(CH₂)₂); 5.08 (t, 1H, C*H*OH), 6.66 (b.s., 1H, OH); 6.98 (d, 1H, H_{3'}, *J =* 4.7 Hz); 7.35-7.44 (m, 2H, H₆+H₅); 7.50-7.57 (m, 2H, H₆,+H₂); 7.69 (t, 1H, H_{7'}); 7.93-8.03 (m, 4H, H₄+H₇+H_{8'}+H_{5'}); 8.62 (d, 1H, H_{2'}, J = 4.6 Hz); 10.07 (b.s., 1H, HCl).

### Example 46:

### 1-(benzo[b]thiophen-3-yl)-3-[4-(quinol-3-yl)piperazin-1-yl]propan-1-ol

The product of the title is made following the general procedure and starting with the ketone synthesised in Example 16.

M.p.: 98-100°C

IR (KBr) (cm⁻¹): 3292 (s, OH).

¹H-NMR (CDCl₃,.₂00 MHz) δ (ppm): ): 2.13 (t, 2H, C*H*_{*2*}CHOH); 2.67-2.91 (m, 6H, N¹(CH₂)₃); 3.36 (t, 4H, N⁴(CH₂)₂); 5.34 (t, 1H, C*H*OH), 7.29-7.52 (m, 5H, H₂+H₅+H₆+H₆'+H₈'); 7.68 (d, 1H, H₅', *J*=6Hz); 7.77-7.86 (m, 2H, H₄+H₄'); 7.98 (d, 1H, H₇, *J*=8Hz); 8.78 (d, 1H, H₂').

### Example 47:

### 1-(benzo[b]thiophen-3-yl)-3-[4-(quinol-6-yl)piperazin-1-yl]propan-1-ol

The product of the title is made following the general procedure and starting with the ketone synthesised in Example 17.

¹H-NMR (CDCl₃, 200 MHz) δ(ppm): 2.02-2.18 (m, 2H, CH₂); 2.60-2.92 (m, 6H, 4H piperazin + CH₂); 3.40 (b.s., 4H, piperazin); 5.39 (t, 1H, CH); 6.62 (b.s., 1H, OH); 7.01 (d, 1H); 7.24-7.54 (b.s., 5H); 7.80-8.04 (b.s., 4H); 8.76 (d, 1H).

### Example 48:

### 6-[4-(3-benzo[b]thiophen-3-yl-3-hydroxypropyl)pipe-razin-1-yl]-1H-indol-2-carboxylic acid, methyl ester

The product of the title is made following the general procedure and starting with the ketone synthesised in Example 18.

¹H-NMR (CDCl₃, 200 MHz) δ(ppm): 2.04-2.18 (m, 2H, CH₂); 2.70-3.00 (m, 6H, 4H piperazine + CH₂); 3.32 (b.s., 4H, piperazine); 3.96 (s, 3H, OCH₃); 5.36-5.42 (m, 1H, CH); 6.59 (d, 1H); 7.04 (d, 1H); 7.20-7.42 (s.c., 4H); 7.44 (s, 1H); 7.80-7.94 (s.c., 2H); 9.01 (b.s., 1H, NH).

### Example 49:

### 4-[4-(3-benzo[b]thiophen-3-yl-3-hydroxypropyl)-piperazin-1-yl]-1H-indol-2-carboxylic acid, methyl ester

The product of the title is made following the general procedure and starting with the ketone synthesised in Example 19.

¹H-NMR (CDCl₃, 200 MHz) δ(ppm): 2.02-2.18 (m, 2H, CH₂); 2.62-2.90 (m, 6H, 4H piperazine + CH₂); 3.26 (b.s., 4H, piperazine); 3.90 (s, 3H, OCH₃); 5.39 (t, 1H, OH); 6.78 (s, 1H); 6.90 (dd, 1H); 7.12 (s, 1H); 7.30-7.40 (s.c., 2H); 7.42 (s, 1H); 7.56 (d, 1H); 7.80-7.92 (s.c., 2H); 8.92 (b.s., 1H, NH).

### Example 50:

### 1-(5-hydroxybenzo[b]thiophen-3-yl)-3-[4-(2,3-dihydro-1,4-benzodioxyn-5-yl)piperazin-1-yl]propan-1-ol

The product of the title is made following the general procedure and starting with the ketone synthesised in Example 23.

M.p.: 168-170°C

IR (KBr) (cm⁻¹): 3413 (m, OH)

¹H-NMR (CDCl₃, 200 MHz) δ (ppm): 1.23 (t, 2H, CHOHC*H*_{*2*}); 2.71 (b.s., 6H, N¹(CH₂)₃); 3.00 (b.s., 4H, N⁴(CH₂)₂); 4.27 (d, 2H, O-(CH₂)₂-O); 5.16 (t, 1H, CHOH); 6.45 (d, 1H, H₆'); 6.55 (d, 1H, H₈'); 6.74 (t, 1H, H₇'); 6.87 (d, 1H, H₆, J₄₆= 6.87); 7.22 (d, 1H, H₄, J= 7.22); 7.35(s, 1H, H₂); 7.60 (d, 1H, H₇, J= 7.60).

### Example 51:

### 1-Benzo[b]thiophen-3-yl-3-[4-(2,3-dihydro-benzofuran-7-yl)-piperazin-1-yl]-propan-1-ol

The product of the title is made following the general procedure and starting with the ketone synthesised in Example 25.

¹H-NMR (CDCl₃, 200 MHz, δ ppm) : 7.90-7.76 (m, 2H); 7.42 (s, 1H); 7.40-7.30 (m, 2H); 6.90-6.65 (m, 3H); 5.39 (t, 1H, CH); 4.62 (t, 2H, OCH2); 3.30-3.10 (m, 6H); 2.90-2.60 (m, 6H); 2.10-2.00 (m, 2H, CH2).

IR: 2.820 cm⁻¹, 1490 cm⁻¹, 1460 cm⁻¹.

### Example 52:

### 1-Benzo[b]thiophen-3-yl-3-(4-benzo[b]-thiophen-5-yl-piperazin-1-yl)-propan-1-ol hydrochloride

The product of the title is made following the general procedure and starting with the ketone synthesised in Example 26.

M.p.: 181-183°C

¹H-NMR (DMSO-d₆ .200 MHz) δ(ppm): 2.23-2-38 (m, 2H, C*H*_{*2*}CHOH); 3.18-3.35 (m, 6H, N¹(CH₂)₃); 3.60-83 (b.s., 4H, N⁴(CH₂)₂); 5.08 (dd, 1H, C*H*OH), 7.17 (dd, 1H, H6'); 7.32-7.41 (m, 5H, H₅+H₆+H_{2'}+H_{4'}+H_{6'}); 7.65 (s, 1H, H₂); 7.70 (d, 1H, H_{3'}, *J*=5.4Hz); 7.86 (d, 1H, H_{7'}); 7.97-8.01 (m, 2H, H₄ +H₇); 10.99 (b.s., 1H, HCl).

### Example 53:

### 1-(5-aminobenzo[b]thiophen-3-yl)-3-[4-(2,3-dihydro-1,4-benzodioxyn-5-yl)piperazin-1-yl]propan-1-ol

To 200 mg of 1-(5-nitrobenzo[b]thiophen-3-yl)-3-[4-(2,3-dihydro-1,4-benzodioxyn-5-yl)piperazin-1-yl]propan-1-ol, made according to Example 37, in 20 ml of MeOH, are added 100 mg of Ni-Raney and 0.5 ml of NH₂NH₂.H₂O. After 2 hours of reaction the mixture is filtered over celite. It is eliminated to dryness. (Yield:85%).

M.p.: 122-125°C

¹H-NMR (CDCl₃, 200 MHz) δ (ppm) : 2.22 (t, 2H, CHOHC*H*_{*2*}), 2.71-2.81 (m, 6H, N¹(CH₂)₃); 3.12 (b.s., 4H, N⁴(CH₂)₂); 4.24 (C, 4H, O(CH₂)₂); 5.25 (t, 1H, CHOH); 6.58-6.68 (m, 2H, H₆'+H₈'); 6.84 (t, 1H, H₇'); 7.09 (dd, 1H, H₇); 7.24 (s, 1H, H₂); 7.45-7.51 (m, 1H, H₆); 7.76 (c, 1H, H₄).

### EXAMPLES OF PHARMACOLOGICAL RESULTS

### Description of the methods used for assessing the pharmacologogical properties

The pharmacological activity of the products of this invention was tested under various experimental models.

### Tests for fixing to serotonin 5HT_{1A} receptors

The technique described by Hoyer and collaborators (Eur. J. Pharmacol. 118:13-23.1985) was used to determine the affinity of the products for the 5-HT_{1A} receptors. The studies were carried out with the fraction of membranes isolated from the cerebral cortex of rats. The agonist ³H-Dipropylaminotetraline (DPAT) was used as specific ligand, while non-radioactive DPAT was used as displacing ligand.

In order to isolate the fraction of membranes, the frontal cortex of the rat was dissected and homogenised in Tris-HCl 50 mM, pH 7.7 at 4°C. The resulting homogenate was centrifuged at 25000 rpm for 15 minutes at 4° C and the sediment resuspended in Tris-HCl containing CaCl₂ 4 mM. For the fixing test the suspension of membranes was incubated with ³H-DPAT (1 nM) and different concentrations of the cold displacing agent. Once the incubation period of 15 minutes at 37 °C had elapsed, the membrane fraction was separated by means of rapid filtration and the radioactivity of the combined fraction quantified by means of liquid scintillation.

### Tests of fixing to the serotonin transporter (5-HT)

The technique described by Marcusson and colleagues (J. Neurochemistry 44: 705-711.1985) was used in order to determine the affinity of the products for the serotonin transporter.

The biological material used was a fraction of semi-purified membranes of the cerebral cortex of rat, obtained using the method described above.

The membrane fraction was resuspended in Tris-HCl 50 mM, pH 7.4 at 4° C, which contained NaCl 120 mM and KCl 5 mM, and was incubated with ³H-paroxetine 0.1 nM and different concentrations of fluoxetine as displacing agent. At the end of the period of incubation of 60 minutes at 22°C the membrane fraction was separated by rapid filtration and the radioactivity of the combined fraction quantified by means of liquid scintillation.

Table I below shows the results of the pharmacological activity of various examples of the product of the invention. The results are expressed as the Kᵢ of the serotonin transporter and the Kᵢ of the 5-HT_{1A} receptor.

**Table 1**

| **EXAMPLE** **No.** | **5-HT** **Ki (nM)** **transporter** | **5-HT1A** **Ki (nM)** |
|---|---|---|
| 21 | 15.9 | 3.2 |
| 27 | 7.5 | 2.7 |
| 28 | 14.9 | 6 |
| 29 | 7.3 | 5.9 |
| 30 | 8.5 | 2.5 |
| 33 | 8.6 | 1.5 |
| 35 | 4.1 | 5.5 |
| 36 | 9.9 | 10.2 |
| 37 | 12.7 | 26.1 |
| 39 | 6.9 | 14 |
| 44 | 6.4 | 377 |
| 45 | 19.4 | 301 |
| 47 | 2.2 | 546 |
| 50 | 6.2 | 18.2 |

## Claims

1. Compound of general formula (I): 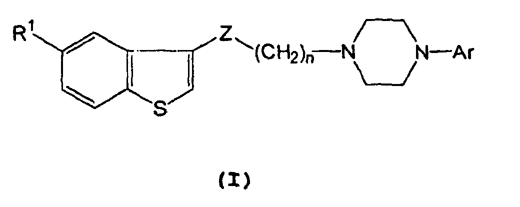 where:
• n is 1, 2 or 3;
• Z is C=O or -CHOH;
• R¹ is H, alkyl C₁-C₆, halogen, -OR², nitro, cyano, -NR³R⁴, -COR², -CO₂R², -O-COR², -SO₂NR³R⁴, -SO₂R², -SR², or -CONR³R⁴;
• R² is H, alkyl C₁-C₆ or phenyl;
• R³ and R⁴ are independent of each other and stand for H, alkyl C₁-C₆ or phenyl, or else R³ together with R⁴ form a morpholine, thiomorpholine or piperazine ring;
• Ar is an optionally substituted bicylic system formed by a benzocondensed heterocyclic ring, which heterocyclic ring has 5, 6 or 7 ring atoms, saturated or unsaturated and containing 1, 2 or 3 hetero-atoms selected from N, O and S;
a pharmaceutically acceptable salt or solvate, or any geometric isomer, optical isomer or polymorph thereof.

2. Compound as claimed in Claim 1, **characterised in that** said heterocyclic ring contains 1 or 2 hetero-atoms selected from N, O and S.

3. Compound as claimed in Claim 1, **characterised in that** Ar is: 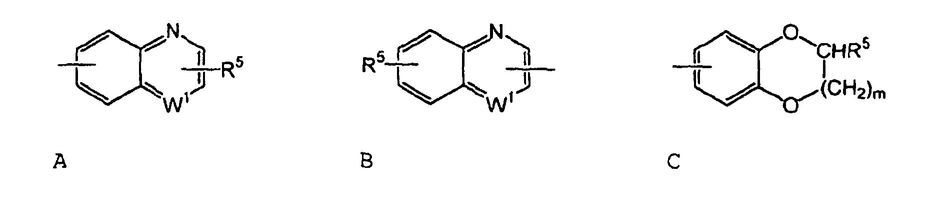 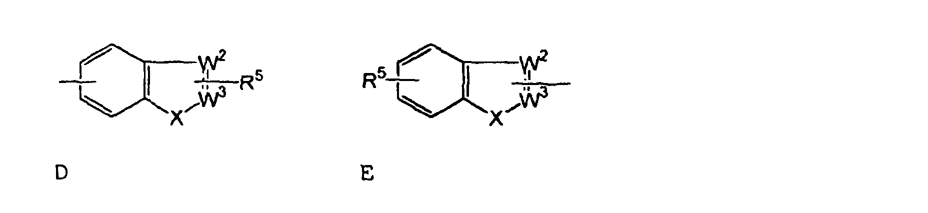 where:
• m is 0, 1 or 2;
• W¹ is CH or N;
• W² and W³ are independently, the same or different, CH, CH₂ or N;
• X is N, O or S;
• R⁵ is H, alkyl C₁-C₆, alkyl C₁-C₆ substituted by a hydroxy or halogen group, halogen, -OR⁶, nitro, cyano, NR⁷R⁸, -COR⁶, -CO₂R⁶, -SO₂NR⁷R⁸, -SO₂R⁶, -SR⁶, or -CONR⁷R⁸ ;
• R⁶ is H, alkyl C₁-C₆ or phenyl;
• R⁷ and R⁸ are independent of each other and stand for H, alkyl C₁-C₆ or phenyl, or else R⁷ together with R⁸ form a morpholine ring, thiomorpholine or piperazine ring.

4. Compound as claimed in Claim 1, **characterised in that** n is 2.

5. Compound as claimed in Claim 1, **characterised in that** R¹ is halogen, NO₂, OH, H or CH₃.

6. Compound as claimed in Claims 4 and 5, **characterised in that** the compound is selected from one of the following:
- 1-(5-nitrobenzo[b]thiophen-3-yl)-3-[4-(2,3-dihydro-1,4-benzodioxyn-5-yl)piperazin-1-yl]propan-1-one
- 1-(5-fluorobenzo[b]thiophen-3-yl)-3-[4-(quinol-8-yl) piperazin-1-yl]propan-1-ol
- 1-(5-fluorobenzo[b]thiophen-3-yl)-3-[4-(2,3-dihydro-1,4-benzodioxyn-5-yl)piperazin-1-yl]propan-1-ol
- 1-(5-fluorobenzo[b]thiophen-3-yl)-3-[4-(indol-4-yl)piperazin-1-yl]propan-1-ol
- 1-(5-fluorobenzo[b]thiophen-3-yl)-3-[4-(quinaldin-8-yl)piperazin-1-yl]propan-1-ol
- 1-(benzo[b]thiophen-3-yl)-3-[4-(quinol-8-yl)piperazin-1-yl]propan-1-ol hydrochloride
- 1-(benzo[b]thiophen-3-yl)-3-[4-(indol-4-yl)piperazin-1-yl]propan-1-ol hydrochloride
- 1-(benzo[b]thiophen-3-yl)-3-[4-(quinaldin-8-yl)piperazin-1-yl]propan-1-ol hydrochloride
- 1-(benzo[b]thiophen-3-yl)-3-[4-(quinol-5-yl)piperazin-1-yl]propan-1-ol
- 1-(5-nitrobenzo[b]thiophen-3-yl)-3-[4-(2,3-dihydro-1,4-benzodioxyn-5-yl)piperazin-1-yl]propan-1-ol
- 1-(benzo[b]thiophen-3-yl)-3-[4-(quinol-2-yl)piperazin-1-yl]propan-1-ol
- 1-(benzo[b]thiophen-3-yl)-3-[4-(quinol-4-yl)piperazin-1-yl]propan-1-ol hydrochloride
- 1-(benzo[b]thiophen-3-yl)-3-[4-(quinol-6-yl)piperazin-1-yl]propan-1-ol
- 1-(5-hydroxybenzo[b]thiophen-3-yl)-3-[4-(2,3-dihydro-1,4-benzodioxyn-5-yl)piperazin-1-yl]propan-1-ol

7. Process for preparing a compound of general formula (I) as claimed in Claim 1, **characterised in that** it comprises:
a substitution reaction of the Hal group of a compound of general formula (II) with a suitable piperazine of general formula (III): 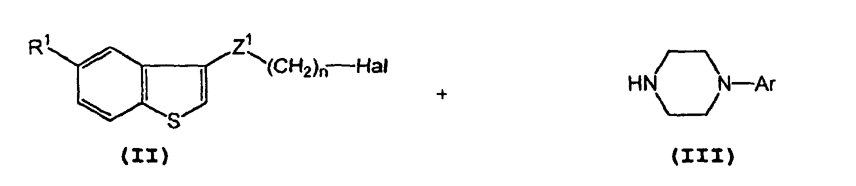 where:
• n, R¹ and Ar have the meaning defined above,
• Hal represents a halogen,
• Z¹ represents C=O, or CHOR⁹ and
• R⁹ represents H or an alcohols protecting group;
in the presence of an inert solvent and an organic or inorganic base, and then, if required, carrying out one or more of the following optional steps:
- Converting a compound of general formula (I) into another compound of general formula (I);
- Eliminating any protecting group;
- Preparing a pharmacologically acceptable salt of a compound of general formula (I) and/or a pharmacologically acceptable solvate thereof.

8. Process as claimed in Claim 7, **characterised in that** said inert solvent is selected from tetrahydrofuran, dichloromethane, toluene or dimethylformamide.

9. Process as claimed in Claim 7, **characterised in that** said organic or inorganic base is selected from potassium bicarbonate, bipotassium bicarbonate, triethylamine and diisopropylamine.

10. Process for preparing a compound of general formula (I) as claimed in Claim 1, where Z=CHOH (Ib), **characterised in that** it is carried out by reduction of a compound of general formula (Ia) where Z is C=O,
where n, R¹ and Ar have the meaning defined above, 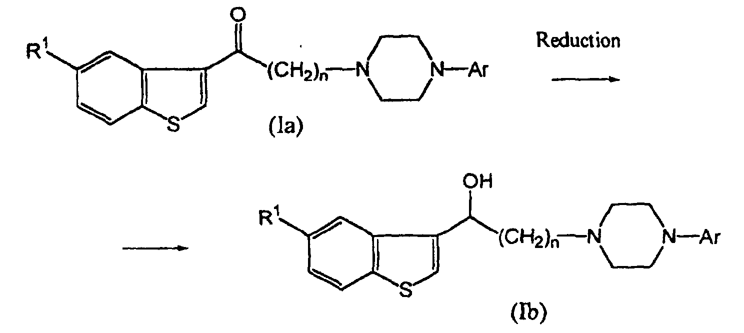
a) in the presence of a reducing agent at a temperature of between -20°C and 200°C and in an inert solvent; or
b) by catalytic hydrogenation;
and then, if required, carrying out one or more of the following optional steps:
- Converting a compound of general formula (I) into another compound of general formula (I);
- Eliminating any protecting group;
- Preparing a pharmacologically acceptable salt of a compound of general formula (I) and/or a pharmacologically acceptable solvate thereof.

11. Process as claimed in Claim 10, **characterised in that** said reducing agent is a metal hydride.

12. Process as claimed in Claim 11, **characterised in that** said metal hydride is sodium borohydride.

13. Process as claimed in Claim 10, **characterised in that** said solvent is an alcohol.

14. Process as claimed in Claim 13, **characterised in that** said alcohol is methyl or ethyl alcohol.

15. Process as claimed in Claims 10 and 13, **characterised in that** said temperature is between -20°C and the reflux temperature of the alcohol, preferably at the reflux temperature.

16. Compound as claimed in any of Claims 1 to 6 for use as serotonin reuptake inhibitor and antagonist or agonist of the 5-HT_{1A} receptor.

17. Use of a compound as claimed in any of Claims 1 to 6, for the manufacturing of a medicine for the treatment of neurological disorders, such as depression, psychosis, anxiety, panic attacks, obsessive-compulsive disorders and nutrition disorders.

18. Pharmaceutical containing a compound as claimed in any of Claims 1 to 6, in a therapeutically active quantity and a suitable quantity of a pharmaceutically acceptable carrier for use as a serotonin reuptake inhibitor and antagonist or agonist of the 5-HT_{1A} receptor.

## Patentansprüche

1. Verbindung der allgemeinen Formel (I): 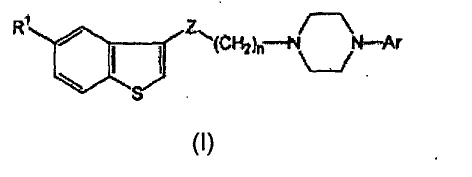 wobei:
- n 1, 2 oder 3 ist;
- Z C=O oder -CHOH ist;
- R¹ H, Alkyl, C₁-C₆, Halogen, -OR², Nitro, Cyano, -NR³R⁴, -COR², -CO₂R², -O-COR², -SO₂NR³R⁴, -SO₂R², -SR² oder -CONR³R⁴ ist;
- R² H, Alkyl, C₁-C₆ oder Phenyl ist;
- R³ und R⁴ unabhängig voneinander sind und für H, Alkyl, C₁-C₆ oder Phenyl stehen, oder andernfalls R³ zusammen mit R⁴ einen Morpholin-, Thiomorpholin- oder Piperazinring bildet;
- Ar ein wahlweise substituiertes bicyclisches System ist, das durch einen benzokondensierten heterocyclischen Ring gebildet wird, wobei der heterocyclische Ring 5, 6 oder 7 Ringatome besitzt, die gesättigt oder ungesättigt sind, und 1, 2 oder 3 Heteroatome, ausgewählt unter N, O und S enthalten;
ein pharmazeutisch verträgliches Salz oder Solvat, oder irgendein geometrisches Isomer, optisches Isomer oder Polymorph davon.

2. Verbindung nach Anspruch 1, **dadurch gekennzeichnet, dass** der heterocyclische Ring 1 oder 2 Heteroatome, ausgewählt unter N, O und S, enthält.

3. Verbindung nach Anspruch 1, **dadurch gekennzeichnet, dass** Ar: 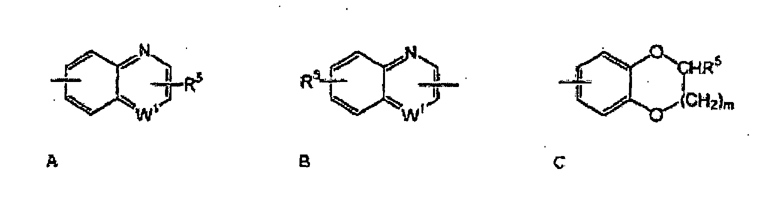 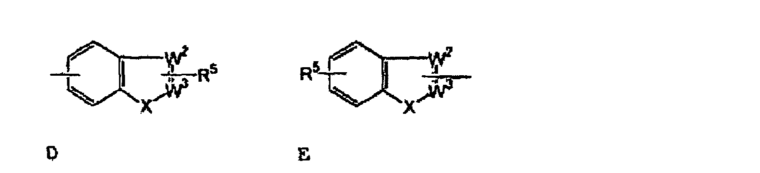 ist, wobei:
- m 0, 1 oder 2 ist;
- W¹ CH oder N ist;
- W² und W³ unabhängig, gleich oder verschieden, CH, CH₂ oder N sind;
- X N, O oder S ist;
- R⁵ H, Alkyl C₁-C₆, Alkyl C₁-C₆ substituiert durch eine Hydroxy- oder Halogengruppe, Halogen, -OR⁶, Nitro, Cyano, NR⁷R⁸, -COR⁶, -CO₂R⁶, -SO₂NR⁷R⁸, -SO₂R⁶, -SR⁶ oder -CONR⁷R⁸ ist;
- R⁶ H, Alkyl C₁-C₆ oder Phenyl ist;
- R⁷ und R⁸ unabhängig voneinander sind und für H, Alkyl C₁-C₆ oder Phenyl stehen, oder andernfalls R⁷ zusammen mit R⁸ einen Morpholinring, Thiomorpholin- oder Piperazinring bilden.

4. Verbindung nach Anspruch 1, **dadurch gekennzeichnet, dass** n 2 ist.

5. Verbindung nach Anspruch 1, **dadurch gekennzeichnet, dass** R¹ Halogen, NO₂, OH, H oder CH₃ ist.

6. Verbindung nach den Ansprüchen 4 und 5, **dadurch gekennzeichnet, dass** die Verbindung eine ist, die aus den Folgenden ausgewählt:
- 1-(5-Nitrobenzo[b]thiophen-3-yl)-3-[4-(2,3-dihydro-1,4-benzodioxin-5-yl) piperazin-1-yl]propan-1-on
- 1-(5-Fluorbenzo[b]thiophen-3-yl)-3-[4-(chinon-8-yl)piperazin-1-yl]propan-1-ol
- 1-(5-Fluorbenzo[b]thiophen-3-yl)-3-[4-(2,3-dihydro-1,4-benzodioxin-5-yl) piperazin-1-yl]propan-1-ol
- 1-(5-Fluorbenzo[b]thiophen-3-yl)-3-[4-(indol-4-yl)piperazin-1-yl]propan-1-ol
- 1-(5-Fluorbenzo[b]thiophen-3-yl)-3-[4-(chinaldin-8-yl)piperazin-1-yl]propan-1-ol
- 1-(Benzo[b]thiophen-3-yl)-3-[4-(chinon-8-yl)piperazin-1-yl]propan-1-ol hydrochlorid
- 1-(Benzo[b]thiophen-3-yl)-3-[4-(indol-4-yl)piperazin-1-yl]propan-1-ol hydrochlorid
- 1-(Benzo[b]thiophen-3-yl)-3-[4-(chinaldin-8-yl)piperazin-1-yl]propan-1-ol hydrochlorid
- 1-(Benzo[b]thiophen-3-yl)-3-[4-chinon-5-yl)piperazin-1-yl]propan-1-ol
- 1-(5-Nitrobenzo[b]thiophen-3-yl)-3-[4-(2,3-dihydro-1,4-benzodioxin-5-yl) piperazin-1-yl]propan-1-ol
- 1-(Benzo[b]thiophen-3-yl)-3-[4-chinon-2-yl)piperazin-1-yl]propan-1-ol
- 1-(Benzo[b]thiophen-3-yl)-3-[4-chinon-4-yl)piperazin-1-yl]propan-1-ol hydrochlorid
- 1-(Benzo[b]thiophen-3-yl)-3-[4-chinon-6-yl)piperazin-1-yl]propan-1-ol
- 1-(5-Hydroxybenzo[b]thiophen-3-yl)-3-[4-(2,3-dihydro-1,4-benzodioxin-5-yl)piperazin-1-yl]propan-1-ol.

7. Verfahren zur Herstellung einer Verbindung der allgemeinen Formel (I) nach Anspruch 1, **dadurch gekennzeichnet, dass** es umfasst:
eine Substitutionsreaktion der Hal Gruppe einer Verbindung der allgemeinen Formel (II) mit einem geeigneten Piperazin der allgemeinen Formel (III): 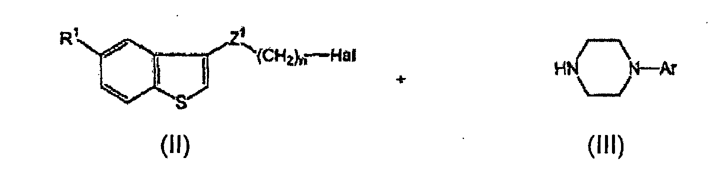
wobei:
- n, R¹ und Ar die oben definierte Bedeutung besitzen,
- Hal ein Halogen dargestellt,
- Z¹ C=O, oder CHOR⁹ darstellt, und
- R⁹ H oder eine Alkoholschutzgruppe darstellt;
in Gegenwart eines inerten Lösungsmittels und einer organischen oder anorganischen Base, und anschließend, falls notwendig, Durchführen eines oder mehrerer der folgenden wahlweisen Schritte:
- Umwandeln einer Verbindung der allgemeinen Formel (I) in eine andere Verbindung der allgemeinen Formel (I);
- Abspalten irgendwelcher Schutzgruppen;
- Herstellen eines pharmakologisch verträglichen Salzes einer Verbindung der allgemeinen Formel (I) und/oder eines pharmakologisch verträglichen Solvates davon.

8. Verfahren nach Anspruch 7, **dadurch gekennzeichnet, dass** das inerte Lösungsmittel ausgewählt ist unter Tetrahydrofuran, Dichlormethan, Toluol oder Dimethylformamid.

9. Verfahren nach Anspruch 7, **dadurch gekennzeichnet, dass** die organische oder anorganische Base ausgewählt ist unter Kaliumbicarbonat, Bikaliumbicarbonat, Triethylamin und Diisopropylamin.

10. Verfahren zur Herstellung einer Verbindung der allgemeinen Formel (I) nach Anspruch 1, wobei Z=CHOH (Ib), **dadurch gekennzeichnet, dass** das Verfahren durchgeführt wird durch Reduktion einer Verbindung der allgemeinen Formel (la), wobei Z C=O ist,
wobei n, R¹ und Ar die oben beschriebene Bedeutung besitzen, 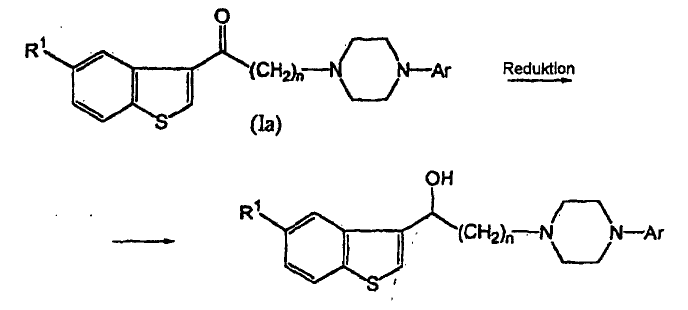
a) in der Gegenwart eines reduzierenden Mittels bei einer Temperatur von zwischen -20°C und 200°C und in einem inerten Lösungsmittel; oder
b) durch katalytische Hydrierung;
und anschließend, falls notwendig, Durchführen eines oder mehrerer der folgenden wahlweisen Schritte:
- Umwandeln einer Verbindung der allgemeinen Formel (I) in eine andere Verbindung der allgemeinen Formel (I);
- Abspalten irgendwelcher Schutzgruppen;
- Herstellen eines pharmakologisch verträglichen Salzes einer Verbindung der allgemeinen Formel (I) und/oder eines pharmakologisch verträglichen Solvates davon.

11. Verfahren nach Anspruch 10, **dadurch gekennzeichnet, dass** das reduzierende Mittel ein Metallhydrid ist.

12. Verfahren nach Anspruch 11, **dadurch gekennzeichnet, dass** das Metallhydrid Natriumborhydrid ist.

13. Verfahren nach Anspruch 10, **dadurch gekennzeichnet, dass** das Lösungsmittel ein Alkohol ist.

14. Verfahren nach Anspruch 13, **dadurch gekennzeichnet, dass** der Alkohol Methyl- oder Ethylalkohol ist.

15. Verfahren nach den Ansprüchen 10 und 13, **dadurch gekennzeichnet, dass** die Temperatur zwischen -20°C und der Rückflusstemperatur des Alkohols liegt, vorzugsweise bei der Rückflusstemperatur.

16. Verbindung nach einem der Ansprüche 1 bis 6, zur Verwendung als Serotoninwiederaufnahmeinhibitor und Antagonist oder Agonist des 5-HT_{1A} Rezeptors.

17. Verwendung einer Verbindung nach einem der Ansprüche 1 bis 6, zur Herstellung eines Arzneimittels für die Behandlung einer neurologischen Störung, wie Depression, Psychose, Angst, Panikattacken, zwanghaften Störungen und Ernährungsstörungen.

18. Pharmazeutikum, enthaltend eine Verbindung nach einem der Ansprüche 1 bis 6, in einer therapeutisch aktiven Menge und einer geeigneten Menge eines pharmazeutisch verträglichen Trägers zur Verwendung als ein Serotoninwiederaufnahmeinhibitor und Antagonist oder Agonist des 5-HT_{1A} Rezeptors.

## Revendications

1. Composé de la formule générale (I) : 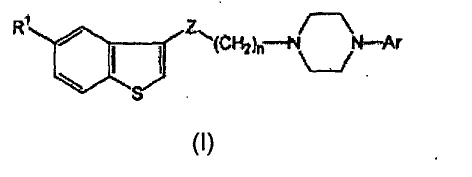 où:
* n est 1, 2 ou 3 ;
* Z est C=O ou -CHOH ;
* R1 est H, alkyl C₁-C₆, halogène, -OR², nitro, cyano, -NR³R⁴, -COR², -CO₂R², -O-COR², -SO₂NR³R⁴, -SO₂R², -SR², ou -CONR³R⁴ ;
* R² est H, alkyl C₁-C₆, ou phényl ;
* R³ et R⁴ sont indépendants l'un de l'autre et représentent H, alkyl C₁-C₆, ou phényl, ou alors R³ avec R⁴ forment un anneau morpholine, thiomorpholine ou pipérazine ;
* Ar est un système bicyclique optionnellement substitué formé par un anneau hétérocyclique benzocondensé, anneau hétérocyclique qui a 5, 6 ou 7 atomes annulaires, saturés ou non et contenant 1, 2 ou 3 hétéro-atomes choisis à partir de N, O et S ;
Un sel ou solvate pharmaceutiquement acceptable, ou tout isomère géométrique, isomère optique ou granulocyte de celui-ci.

2. Composé selon la revendication 1, **caractérisé en ce que** ledit anneau hétérocyclique contient 1 ou 2 hétéro-atomes sélectionnés à partir de N, O et S.

3. Composé selon la revendication 1, **caractérisé en ce que** Ar est : 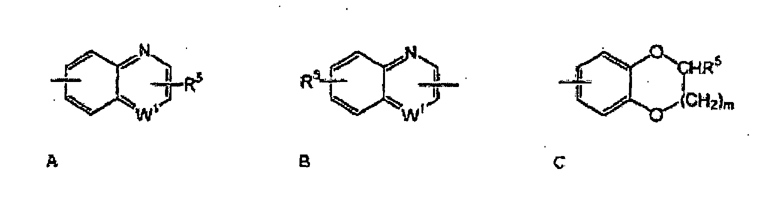 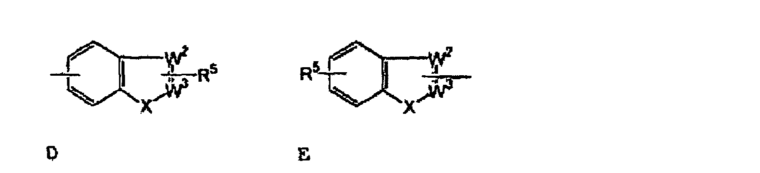 Où :
* m est 0, 1 ou 2 ;
* W¹ est CH ou N ;
* W² et W³ sont indépendamment identiques ou différents, CH, CH₂ ou N ; X est N, O ou S ;
* R⁵ est H, alkyl C₁-C₆, alkyl C₁-C₆ substitué par un groupe hydroxy ou halogène, halogène, -OR⁶, nitro, cyano, -NR⁷R⁸, -COR⁶, -CO₂R⁶, -SO₂NR⁷R⁸, -SO₂R⁶, -SR⁶, ou -CONR⁷R⁸;
* R⁶ est H, alkyl C₁-C₆, ou phényl ;
* R⁷ et R⁸ sont indépendants l'un de l'autre et représentent H, alkyl C₁-C₆,ou phényl,
ou alors R⁷ avec R⁸ forment un anneau morpholine, thiomorpholine ou pipérazine ;

4. Composé selon la revendication 1, **caractérisé en ce que** n est 2.

5. Composé selon la revendication 1, **caractérisé en ce que** R¹ est halogène, NO₂, OH, H ou CH₃.

6. Composé selon les revendications 4 et 5, **caractérisé en ce que** le composé est choisi parmi l'un des suivants :
- 1-(5-nitrobenzo[b]thiophène-3-yl)-3-[4-(2,3-dihydro-1,4-benzodioxyne-5-yl)pipérazine-1-yl]propane-1-un
- 1-(5-fluorobenzo[b]thiophène-3-yl)-3-[4-(quinol-8-yl)pipérazine-1-yl] propane-1-ol
- 1-(5-fluorobenzo[b]thiophène-3-yl)-3-[4-(2,3-dihydro-1,4-benzodioxyne-5-yl)pipérazine-1-yl]propane-1-ol
- 1-(5-fluorobenzo[b]thiophène-3-yl)-3-[4-(indol-4-yl)pipérazine-1-yl] propane-1-ol
- 1-(5-fluorobenzo[b]thiophène-3-yl)-3-[4(quinaldine-8-yl)pipérazine-1-yl] propane-1-ol
- 1-(benzo[b]thiophène-3-yl)-3-[4-quinol-8-yl)pipérazine-1-yl)propane-1-ol chlorhydrate
- 1-(benzo[b]thiophène-3-yl)-3-[4-(indol-4-yl)pipérazine-1-yl]propane-1-ol chlorhydrate
- 1-(benzo{b]thiophène-3-yl)-3-[4-(quinaldine-8-yl)pipérazine-1-yl] propane-1-ol chlorhydrate
- 1-(benzo[b]thiophène-3-yl)-3-[4-(quinol-5-yl)pipérazine-1-yl]propane-1-ol
- 1-(5-nitrobenzo[b]thiophène-3-yl)-3-[4-(2,3-dihydro-1,4-benzodioxyne-5-yl)pipérazine-1-yl]propane-1-ol
- 1-(benzo[b]thiophène-3-yl)-3-[4-(quinol-2-yl)pipérazine-1-yl]propane-1-ol
- 1-(benzo[b]thiophène-3-yl)-3-[4-(quinol-4-yl)pipérazine-1-yl]propane-1-ol chlorhydrate
- 1-(benzo[b]thiophène-3-yl)-3-[4-(quinol-6-yl)pipérazine-1-yl]propane-1-ol
- 1-(5(hydroxybenzo[b]thiophène-3-yl)-3-[4-(2,3-dihydro-1,4-benzodioxyne-5-yl)pipérazine-1-yl]propane-1-ol

7. Procédé pour préparer un composé de la formule générale (I) selon revendication 1, **caractérisé en ce qu'**il comprend :
une réaction de substitution du groupe Hal d'un composé de la formule générale (II) avec une pipérazine adéquate de formule générale (III) : 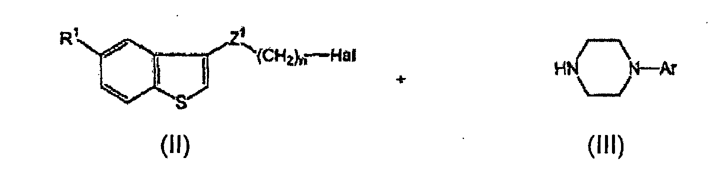
où :
- n, R¹ et Ar ont la signification définie ci-dessus,
- Hal représente un halogène,
- Z¹ représente C=0, ou CHOR⁹ et
- R⁹ représente H ou un groupe protégeant les alcools;
en présence d'un solvant inerte et d'une base organique ou inorganique, et ensuite, si besoin est, la réalisation d'une ou de plusieurs des étapes optionnelles suivantes :
- la conversion d'un composé de la formule générale (I) en un autre composé de la formule générale (I) ;
- l'élimination d'un groupe de protection;
- la préparation d'un sel pharmacologiquement acceptable d'un composé de la formule générale (I) et/ou un solvate pharmacologiquement acceptable de celui-ci.

8. Procédé selon la revendication 7, **caractérisé en ce que** ledit solvant inerte est choisi à partir de tétrahydrofurane, dichlorométhane, toluène ou diméthylformamide.

9. Procédé selon la revendication 7, **caractérisé en ce que** ladite base organique ou inorganique est choisie à partir de bicarbonate de potassium, bicarbonate de bipotassium, triéthylamine et diisopropylamine.

10. Procédé pour la préparation d'un composé de la formule générale (I) selon revendication 1, où Z=CHOH (Ib), **caractérisé en ce qu'**il est réalisé par réduction d'un composé de la formule générale (la) où Z est C=O,
où n, R1 et Ar ont la signification définie ci-dessus, 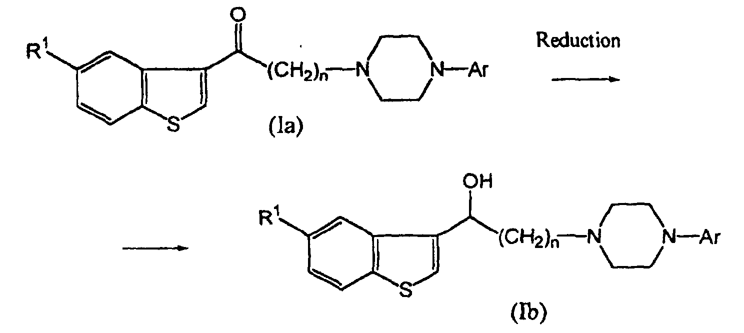
a) en présence d'un agent réducteur à une température entre - 20°C et 200°C et dans un solvant inerte ; ou
b) par hydrogénation catalytique;
et ensuite, si besoin est, la réalisation d'une ou de plusieurs des étapes optionnelles suivantes :
- conversion d'un composé de la formule générale (I) en un autre composé de la formule générale (I);
- élimination d'un groupe de protection;
- préparation d'un sel pharmacologiquement acceptable d'un composé de la formule générale (I) et/ou un solvate pharmacologiquement acceptable de celui-ci.

11. Procédé selon la revendication 10, **caractérisé en ce que** ledit agent réducteur est un hydrure de métal.

12. Procédé selon la revendication 11, **caractérisé en ce que** ledit hydrure de métal est du borohydrure de sodium.

13. Procédé selon la revendication 10, **caractérisé en ce que** ledit solvant est un alcool.

14. Procédé selon la revendication 13, **caractérisé en ce que** ledit alcool est de l'alcool de méthyle ou d'éthyle.

15. Procédé selon les revendications 10 et 13, **caractérisé en ce que** ladite température est entre - 20°C et la température de reflux de l'alcool, de préférence à la température de reflux.

16. Composé selon l'une des revendications 1 à 6 pour une utilisation comme inhibiteur de recaptage de sérotonine et antagoniste ou agoniste du récepteur 5-HT_{1A}.

17. Utilisation d'un composé selon revendication dans l'une des revendications 1 à 6 pour la fabrication d'un médicament pour le traitement des troubles neurologiques, comme la dépression, psychose, anxiété, crises de panique, troubles d'obsession impulsive et troubles de la nutrition.

18. Produit pharmaceutique contenant un composé selon l'une des revendications 1 à 6, en quantité thérapeutiquement active et une quantité appropriée d'un porteur pharmaceutiquement acceptable à utiliser comme inhibiteur de recaptage de sérotonine et antagoniste ou agoniste du récepteur 5-HT_{1A}.
